(19) Europäisches Patentamt
European Patent Office
Office européen des brevets

(11) **EP 4 328 579 B1**

(12) **EUROPÄISCHE PATENTSCHRIFT**

(45) Veröffentlichungstag und Bekanntmachung des Hinweises auf die Patenterteilung:
**28.01.2026  Patentblatt 2026/05**

(21) Anmeldenummer: **23192682.5**

(22) Anmeldetag: **22.08.2023**

(51) Internationale Patentklassifikation (IPC):
**G01N 29/02** (2006.01)

(52) Gemeinsame Patentklassifikation (CPC):
**G01N 29/022; G01N 29/036;** G01N 33/54373;
G01N 2009/006; G01N 2291/0256;
G01N 2291/0427

(54) **SENSOR ZUR UMWANDLUNG CHEMISCHER UND/ODER BIOCHEMISCHER INFORMATION EINES ANALYTEN**

SENSOR FOR CONVERTING CHEMICAL AND/OR BIOCHEMICAL INFORMATION OF AN ANALYTE

CAPTEUR POUR LA CONVERSION D'INFORMATIONS CHIMIQUES ET/OU BIOCHIMIQUES D'UN ANALYTE

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **22.08.2022  DE 102022121188**

(43) Veröffentlichungstag der Anmeldung:
**28.02.2024  Patentblatt 2024/09**

(73) Patentinhaber: **Digid GmbH**
**55129 Mainz (DE)**

(72) Erfinder:
• **KÖNNE, Nils, Dr.**
**55129 Mainz (DE)**
• **VON GERSDORF, Constantin**
**55129 Mainz (DE)**
• **KLOPPSTECH, Konstantin, Dr.**
**55129 Mainz (DE)**
• **KÖHLER, Malte, Dr.**
**55129 Mainz (DE)**

(74) Vertreter: **Nordmeyer, Philipp Werner**
**Maucher Jenkins**
**Patent- und Rechtsanwälte**
**Liebigstraße 39**
**80538 München (DE)**

(56) Entgegenhaltungen:
WO-A1-2005/100965     WO-A1-2017/009868
WO-A1-97/09584     WO-A2-2006/083566
DE-T2- 60 023 917

**Beschreibung**

Technisches Gebiet

Die vorliegende Erfindung betrifft einen Sensor zur Umwandlung chemischer und/oder biochemischer Information eines Analyten in einer Probe in ein elektrisches Signal, um auf diese Weise eine qualitative Aussage über das Vorliegen des Analyten in der Probe und/oder eine quantitative Aussage über den Analyten in der Probe anhand des erzeugten elektrischen Signals abzuleiten.

Stand der Technik

Die Verwendung von Federelementen beziehungsweise Cantilevern zur Detektion von Analyten in Proben ist bekannt. Hierbei wird die Wechselwirkung von Cantilevern mit einer Probenflüssigkeit und die Bindung des Analyten in der Probe an eine Beschichtung des Cantilevers ausgenutzt, um eine Verformung des Cantilevers hervorzurufen. Aus der Verformung kann dann über einen Dehnungsmessstreifen auf das Vorkommen des Analyten geschlossen werden.

Die Verformung von Cantilevern durch unterschiedliche Oberflächenspannungen ist beispielsweise in Rasmussen, P. A., Hansen, O., & Boisen, A. (2005). Cantilever surface stress sensors with single-crystalline silicon piezoresistors. Applied Physics Letters, 86(20), 203502. https://doi.org/10.1063/1.1900299 beschrieben.

Die WO 2007/088018 A1 schlägt ferner Federelemente zur Verwendung in Biosensoren wie beispielsweise der DNA-Analyse vor.

Die Detektion eines Analyten in einer Probe kann sich jedoch aufgrund der großen mechanischen und thermischen Störeinflüssen der Probenflüssigkeit und wegen weiterer chemischer Substanzen in der Probenflüssigkeit als schwierig erweisen. Aus diesem Grund ist es notwendig, aus dem eigentlichen Messsignal die Anteile herauszufiltern, die lediglich auf die Wechselwirkung des Analyten mit dem Cantilever zurückzuführen sind.

Aus DE 600 23 917 T2 sind Sensoren mit einer mikroskopischen, flexiblen, mechanischen Struktur bekannt. Aus WO 97/09584 A sind Cantilever mit Dehnungssensorelementen zur Umwandlung mechanischer Bewegungen des Cantilevers in elektrische Signale bekannt. Aus WO 2005/100965 A1 sind Polymercantilver für Anwendungen in der Biosensorik bekannt. Aus WO 2005/100965 A1 sind Cantilever mit einem Oberflächenspannungssensor bekannt.

Darstellung der Erfindung

Ausgehend von dem bekannten Stand der Technik ist es eine Aufgabe der vorliegenden Erfindung, einen verbesserten Sensor zur Umwandlung chemischer und/oder biochemischer Information bereitzustellen.

Die Aufgabe wird durch einen Sensor zur Umwandlung chemischer und/oder biochemischer Information mit den Merkmalen des Anspruchs 1 gelöst. Vorteilhafte Weiterbildungen ergeben sich aus den Unteransprüchen, der Beschreibung und den Figuren.

Entsprechend wird ein Sensor zur Umwandlung chemischer und/oder biochemischer Information eines Analyten in einer Probe in ein elektrisches Signal vorgeschlagen, umfassend einen Testcantilever, der eine Basis und einen verformbaren Teil aufweist, wobei mindestens auf dem verformbaren Teil eine Rezeptorschicht zur selektiven Aufnahme eines Analyten der Probe aufgebracht ist, wobei auf dem Testcantilever ein erster und ein zweiter Testtransducer angeordnet sind, einen Referenzcantilever, der eine Basis und einen verformbaren Teil aufweist, wobei mindestens auf dem verformbaren Teil eine Referenzschicht zur selektiven Nichtaufnahme des Analyten aufgebracht ist, wobei auf dem Referenzcantilever ein erster und ein zweiter Referenztransducer angeordnet sind, wobei die Transducer dazu ausgebildet und eingerichtet sind, ein dem Vorkommen und/oder der Konzentration des Analyten in der Probe entsprechendes elektrisches Signal auszugeben, wobei durch die selektive Nichtaufnahme des Analyten durch die Referenzschicht die Wechselwirkung des Referenzcantilevers mit der Probe mit dem Analyten der Wechselwirkung des Testcantilevers mit der Probe ohne Analyten entspricht. Erfindungsgemäß sind die Testtransducer auf dem verformbaren Teil des Testcantilevers angeordnet und die Referenztransducer sind auf dem verformbaren Teil des Referenzcantilevers angeordnet.

Eine Probe bezeichnet hierbei eine beschränkte Menge eines Stoffes, die einer größeren Menge des Stoffes, etwa aus einem Reservoir, entnommen wurde, wobei die Zusammensetzung der Probe repräsentativ für die Zusammensetzung des Stoffs in dem Reservoir ist und dementsprechend aus dem Stoffvorkommen und Stoffzusammensetzungen der Probe auf das entsprechende Vorkommen im Reservoir geschlossen werden kann.

Beispielsweise kann eine Probe eine Speichelprobe sein, oder eine Blutprobe sein, oder eine Urinprobe sein, oder ein Abstrich sein, insbesondere ein Rachenabstrich oder ein Nasenabstrich oder ein Nebenhöhlenabstrich sein, oder entnommenes Gewebe sein. Eine Probe umfasst insbesondere jegliche Art von biologischer Probe, als insbesondere auch Proben von Tieren. Eine Probe kann auch eine nichtbiologische Probe, beispielsweise eine Probe eines chemischen Stoffs, sein.

Insbesondere kann eine Probenform in eine weitere Probenform überführt werden, sodass der Analyt, beziehungsweise dessen Vorkommen in einer einfachen und sicheren Art und Weise detektiert werden kann. Beispielsweise kann ein Abstrich in einer Flüssigkeit gelöst werden, sodass der in der Flüssigkeit gelöste Abstrich dann die eigentliche Probe ist. Beispielsweise kann die Probe eine Lymphflüssigkeit beziehungsweise Lymphe sein oder enthalten.

Die Probe enthält dann die chemische Information und/oder biochemische Information über den Analyten. Ein Analyt ist hierbei der Stoff, dessen Vorliegen in der Probe qualitativ und/oder quantitativ nachgewiesen werden soll beziehungsweise mit dem Sensor detektiert werden soll. Der Analyt kann insbesondere unmittelbar in der Probe vorhanden sein, oder in der Probe gelöst sein oder der Probe oder einem Teil der Probe, insbesondere einem Probenpartikel, anhaften. Der Analyt kann mit der Probe auch eine chemische, biologische und/oder physikalische Wechselwirkung eingehen, sodass der Analyt lediglich indirekt über eine entsprechende Wechselwirkung detektierbar ist.

Die chemische Information kann beispielsweise die Art des Analyten, die Konzentration des Analyten, das Vorkommen des Analyten, das Gewicht des Analyten, die Reaktivität des Analyten, die Dichte des Analyten usw. umfassen. Die biochemische Information umfasst dieselben Eigenschaften wie die chemische Information, jedoch können diese Stoffe beispielsweise durch biologische Prozesse entstehen. Insbesondere spricht man von biochemischer Information, wenn der Analyt einen besonderen Einfluss auf den biologischen Kreislauf, beispielsweise den Stoffwechsel oder auf das Immunsystem hat.

Zur Umwandlung der chemischen und/oder biochemischen Information des Analyten in ein elektrisches Signal umfasst der Sensor einen Referenzcantilever und einen Testcantilever. Ein Cantilever ist hierbei ein Federelement, welches eine Basis und einen verformbaren Teil aufweist.

Die Basis ist hierbei ein unbeweglicher Teil des Cantilevers, der insbesondere ortsfest mit einem Substrat verbunden ist und/oder unterstützt wird und/oder aus dem Substrat herausgearbeitet ist. Die Basis der Cantilever ist als starre Basis ausgebildet, so dass lediglich der verformbare Teil des Cantilevers verformbar ausgebildet ist.

Der verformbare Teil des Cantilevers erstreckt sich in Längsrichtung über das Substrat, auf dem die Basis angeordnet ist, hinaus. Mit anderen Worten ist der verformbare Teil des Cantilevers einseitig an der Basis aufgehängt und nicht von dem Substrat unterstützt. Indem der verformbare Teil über das Substrat hinausragt, lässt sich der verformbare Teil des Cantilevers verbiegen, auslenken und dehnen. Die räumliche Grenze, ab der der Cantilever biegbar ist beziehungsweise der Cantilever von der Basis in den verformbaren Teil übergeht, wird Biegekante genannt. Die Biegekante ist üblicherweise eine Kante des Substrats, wenn der Cantilever über das Substrat hinausragt.

Wird der Cantilever verformt, so ergeben sich Materialspannungen und Kräfte in oder auf dem Material des Cantilevers, welche gemessen werden können. Sofern eine solche Materialspannung und/oder Kraft gemessen werden kann, lässt sich darüber auf eine Verformung des Cantilevers schließen. Eine Verformung kann eine anhebende oder absenkende Verformung sein. Der Cantilever kann sich aber auch selbst verformen, beispielsweise aufwölben, wellen oder verzerren.

Die Verformung der Cantilever kann durch eine geeignete Beschichtung stoffspezifisch herbeigeführt werden. Aus diesem Grund weist der Referenzcantilever eine Referenzschicht zur selektiven Nichtaufnahme des Analyten auf, während der Testcantilever eine Rezeptorschicht zur Aufnahme des Analyten aufweist.

Eine Rezeptorschicht ist hierbei ein Stoff, der mit dem Analyten in Wechselwirkung treten kann. Dies bedeutet wiederum, dass die Rezeptorschicht spezifisch für jeden Analyten gewählt wird. Analog ist eine Referenzschicht ein Stoff, der mit dem Analyten nicht in Wechselwirkung treten kann. Auch die Referenzschicht wird daher spezifisch für den Analyten gewählt.

Wechselwirkung bedeutet in diesem Fall, dass der Analyt in chemischer und/oder biochemischer und/oder physikalischer Wechselwirkung mit der Rezeptorschicht steht. Insbesondere kann die Wechselwirkung in einer Bindung des Analysten an die Rezeptorschicht bestehen. Eine Wechselwirkung kann außerdem in der Absorption oder Adsorption oder einer unspezifischen Adhäsion des Analyten an die Rezeptorschicht bestehen.

Die Rezeptor- und Referenzschichten sind bevorzugt chemisch identisch bezüglich möglicher Störeinflüsse und unterscheiden sich bevorzugt nur durch die Wechselwirkung mit dem Analyten. Ein Stoff, der nicht der Analyt ist, wechselwirkt dementsprechend genauso stark oder genauso schwach mit der Rezeptorschicht wie mit der Referenzschicht.

Die selektive Aufnahme des Analyten am Testcantilever bewirkt, dass eine Kraft durch den Analyten auf den Testcantilever wirkt, so dass der Testcantilever sensitiv auf den Analyten reagiert. Dementsprechend tragen die anderen Stoffe der Probe, die nicht der Analyt sind, lediglich zu einem Grundrauschen in Form einer Grundverbiegung am Testcantilever bei. Die Kraft auf den Testcantilever steigt beispielsweise umso schneller größer, je größer die Konzentration des Analyten in der Probe ist oder je schneller die Oberfläche des Cantilevers mit dem Analyten belegt ist. Eine für die jeweilige Ausbildung mögliche Maximalkraft wird bei einer vollständigen Belegung des Cantilevers erreicht.

Die selektive Nichtaufnahme des Analyten am Referenzcantilever bewirkt hingegen, dass keine Kraft durch den Analyten auf den Referenzcantilever wirkt, so dass nur die Stoffe, die nicht der Analyt sind, zu einem Grundrauschen in Form einer Grundverbiegung des Referenzcantilevers beitragen.

Diese wirkende Kraft kann bei dem verformbaren Teil des Testcantilevers eine Verformung bewirken, während der verformbare Teil des Referenzcantilever nicht verbogen wird. Grundlage für die Auslenkung des Cantilevers ist die Änderung der Oberflächenspannung durch die Wechselwirkung mit dem Analyten. Die Änderung der Oberflächenspannung führt zu einer Dehnung oder Stauchung der oberen (oder unteren) Oberfläche des Cantilevers. Die unterschiedliche Dehnung oder Stauchung an Ober- und Unterseite bewirkt in dem Material eine interne Kraft oder Materialspannung, die zur Verformung führt.

Referenzcantilever nach dem Stand der Technik weisen lediglich keine Rezeptorschicht auf, die sensitiv auf den Analyten reagiert. Dadurch können zwar Effekte wie Turbulenz in der Probe und die thermische Drift des Sensorsystems bestimmt werden. Jedoch kann bei einem solchen Referenzcantilever der Analyt beispielsweise durch eine unspezifische Bindung an die Referenzschicht des Referenzcantilevers binden. Dadurch trägt aber der Analyt selbst zum Grundrauschen bei. Daher sind bei einem Sensor nach dem Stand der Technik Referenzmessungen in einer Referenzprobe, also einer Probe ohne Analyten, notwendig. Nur dadurch lässt sich der Effekt der unspezifischen Bindung der Stoffe, die nicht der Analyt sind, feststellen.

Bei dem vorgeschlagenen Sensor wird durch die selektive Nichtaufnahme des Analyten durch den Referenzcantilever das Messverfahren drastisch vereinfacht, da der Referenzcantilever nicht sensitiv für den Analyten ist und daher der Analyt auch nicht zum Grundrauschen beiträgt. Nur die Stoffe, die nicht der Analyt sind, tragen hier zum Grundrauschen des Referenzcantilevers bei. Gewissermaßen kann durch die selektive Nichtaufnahme des Analyten am Referenzcantilever bewirkt werden, dass der Referenzcantilever denselben Turbulenzen, derselben thermischen Drift und demselben Einfluss aller Stoffe, die nicht der Analyt sind, ausgesetzt ist, wie in einer Referenzprobe. Jedoch mit dem Unterschied, dass das Referenzsignal direkt in der Probenflüssigkeit bestimmt wird.

Insbesondere bewirkt ein Referenzcantilever mit Referenzschicht und ein Testcantilever mit Rezeptorschicht eine deutlich spezifischere Analyse des Analyten als lediglich ein Referenzcantilever ohne Rezeptorschicht, da sowohl die Referenzschicht als auch die Rezeptorschicht eine spezifische Wechselwirkung beziehungsweise Nicht-Wechselwirkung mit dem Analyten aufweisen.

Der Aufbau des Sensors mit Referenzcantilever und Testcantilever hat den Vorteil, dass in der Probe gleichzeitig zwei Messungen vorgenommen werden können, wobei die Messung des Referenzcantilevers die Messung des Testcantilevers kalibrieren kann. Dadurch lassen sich Umgebungseinflüsse, etwa chemische, thermische, mechanische, elektrische und fluidische Störeinflüsse, auf die jeweilige Messung reduzieren, so dass ein Vorkommen des Analyten aus dem Vergleich der Messung am Testcantilever und am Referenzcantilever geschlossen werden kann.

Diese Kräfte oder Materialspannungen, beispielsweise Dehnungen oder Stauchungen, die auf die Cantilever wirken, können schließlich von den Transducern detektiert werden, wobei durch unterschiedlich starke Dehnungen oder Stauchungen unterschiedlich starke Spannungen von den Transducer detektiert werden.

Die Transducer haben demnach den Zweck, die Verformung der Cantilever zu bestimmen oder zu messen. Erfindungsgemäß sind die Transducer auf den verformbaren Teilen der Cantilever angeordnet. Beispielsweise kann eine Verformung des Cantilevers dazu führen, dass der Widerstand eines Transducers ansteigt oder abfällt, während keine Verformung des Cantilevers auch keine Veränderung des Widerstands des Transducers hervorruft. Dies kann beispielsweise über eine Ausbildung der Transducer nach dem Prinzip eines Dehnungsmessstreifens erfolgen, wodurch sich eine Verformung des jeweiligen Cantilevers in einer Längenänderung des darauf aufgebrachten Dehnungsmessstreifens des Transducers äußert und damit eine Verformung des Cantilevers direkt durch eine Veränderung des Widerstands des Dehnungsmessstreifens detektiert werden kann.

Somit wird die chemische und/oder biochemische Information des Analyten über einer Verformung des Cantilevers, eine anschließende Registrierung über einen Transducer, und schließlich über eine Änderung einer elektrischen Eigenschaft des Transducers detektierbar.

Indem die ersten und zweiten Testtransducer auf den verformbaren Teilen des Testcantilevers angeordnet sind und die ersten und zweiten Referenztransducer auf dem verformbaren Teil des Referenzcantilevers angeordnet sind, kann ein Maß der Dehnung des Testtransducers gefunden werden, welches der Stärke der Wechselwirkung des Analyten mit dem verformbaren Teil des Testcantilevers entspricht.

Beispielsweise kann der erste Referenztransducer des Referenzcantilevers durch Einfluss der Umgebungsbedingungen und Wechselwirkung mit der Probe einen ersten elektrischen Referenzzustand des ersten Referenztransducers hervorrufen, während die Wechselwirkung des Testcantilevers mit den Umgebungsbedingungen der Probe einen ersten elektrischen Testzustand des ersten Testtransducers hervorruft.

Beispielsweise kann der Referenzcantilever durch den Einfluss der Umgebungsbedingungen um einen ersten Betrag verbogen werden, sodass die Auslenkung in dem ersten Referenztransducer einen ersten elektrischen Referenzzustand hervorruft und im zweiten Referenztransducer einen zweiten elektrischen Referenzzustand hervorruft. Der Testcantilever wird durch den Einfluss der Umgebungsbedingungen um einen zweiten Betrag verbogen und durch die zusätzliche Wechselwirkung mit dem Analyten in der Probe um einen dritten Betrag verbogen wird, was im ersten Testtransducer einen ersten elektrischen Testzustand hervorruft und im zweiten Testtransducer einen zweiten elektrischen Testzustand hervorruft.

Der Vergleich der elektrischen Zustände der ersten und zweiten Transducer geben ein Maß für die Verformung der Cantilever an. Gleichzeitig ergibt ein Vergleich der jeweils ersten Transducer und/oder der jeweils zweiten Transducer ein Maß für die Unterschiedlichkeit der Verformung der Cantilever. Dadurch ist es möglich auf einen spezifischen Einfluss eines Analyten auf den Testcantilever zu schließen.

Die Bauweise mit vier Transducern hat den Vorteil, dass eine solche lokale Kalibrierung des Sensors am Ort des Einflusses der Probe und des Analyten möglich ist.

Die zu detektierende Kraft kann eine Biegekraft und/oder eine Dehnungskraft und/oder eine Scherkraft und/oder eine Stauchungskraft sein und/oder auf der Biegesteifigkeit der Referenzcantilever und Testcantilever beruhen.

Eine Biegekraft kann eine Veränderung der Geometrie des Cantilevers hervorrufen, insbesondere dem Cantilever eine Krümmung aufprägen, die sich vom unbeanspruchten Cantilever unterscheidet.

Eine Dehnungskraft oder Stauchungskraft kann insbesondere eine Längenänderung des Cantilevers hervorrufen. Die jeweilige Längenänderung kann je nach Richtung des Kristallgitters des Cantilevers unterschiedlich sein.

Die Dehnung (oder Stauchung) kann insbesondere an der oberen Oberfläche unterschiedlich zu unteren Oberfläche des Cantilevers sein. Die Dehnung der Oberfläche kann insbesondere parallel zur Basis des Cantilevers erfolgen (eine sogenannte Querdehnung) oder senkrecht zur Basis des Cantilevers (eine sogenannte Längsdehnung). Die Größe der Dehnung hängt hierbei stark von der Geometrie und der Kristallstruktur der Cantilever sowie der weiteren auf den Oberflächen vorgesehenen Schichten, beispielsweise der Elektroden, ab, so dass eine optimale Detektion des Analyten durch eine Optimierung von Ausrichtung und Cantilevergeometrie erreicht werden kann.

Sofern die Dehnungskraft an der oberen Oberfläche und der unteren Oberfläche unterschiedlich ist, bezeichnet man die wirkende Kraft auch als Scherkraft.

Bei einem durchgebogenen Cantilever wirkt eine Biegekraft, da dem Cantilever eine Krümmung aufgeprägt wird. Dadurch wird die obere Oberfläche des Cantilevers gedehnt und diese Dehnung ist insbesondere größer als an der unteren Oberfläche des Cantilevers, sodass insgesamt auch eine Scherkraft auf den Cantilever wirkt.

Die oben genannten Kräfte basieren alle auf dem sogenannten Elastizitätsmodul des Cantilevers. Das Elastizitätsmodul des Cantilevers ist eine Materialkonstante, die spezifisch für das verwendete Material des Cantilevers ist. Durch Wahl des Materials oder der Materialkomposition beziehungsweise durch Bearbeitung des Materials lässt sich das Elastizitätsmodul in einem gewissen Rahmen einstellen, so dass der zu messende Effekt für die jeweils eingerichteten Transducer optimiert werden kann. Umgekehrt ist es natürlich auch möglich die Transducer auf das vorliegende Elastizitätsmodul des Materials anzupassen und deren Empfindlichkeit zu optimieren.

Bevorzugt können die Cantilever sogenannte Bimaterialcantilever sein, beispielsweise Cantilever aus einer Gold- und einer Siliziumnitridschicht. Ein Bimaterialcantilever besteht aus Materialschichten, die zusammen einen definierten Spannungszustand aufweisen. Beispielsweise kann der Zustand spannungsfrei sein, so dass die intrinsischen mechanischen Spannungen minimal sind. Es kann aber auch sein, dass ein Bimaterialcantilever vorgespannt ist, so dass der Cantilever besonders sensitiv auf eine Änderung der Oberflächenspannung reagiert. Es kann aber auch sein, dass ein homogener Cantilever auf der Oberseite und der Unterseite unterschiedlich beschichtet wird, um den beschriebenen Bimaterialeffekt zu imitieren. Insbesondere können isotrope und anisotrope Materialien für die Cantilever verwendet werden, um so eine Optimierung der longitudinalen und transversalen Dehnung zu erreichen. Beispielsweise kann ein isotropes Metall auf einem anisotropen Substrat verwendet werden.

Die verformbaren Teile der Referenz- und Testcantilever können identische geometrische Abmessungen aufweisen, wobei bevorzugt die Breite des verformbaren Teils der Referenz- und Testcantilever der Länge des verformbaren Teils der Referenz- und Testcantilever entspricht, wobei besonders bevorzugt die verformbaren Teile der Referenz- und Testcantilever weniger als $200\,\mu m$ breiter, weniger als $200\,\mu m$ lang und weniger als $1\,\mu m$ dick sind, insbesondere $50\,\mu m$ breit, $50\,\mu m$ lang und $0,3\,\mu m$ dick sind oder $125\,\mu m$ breit, $20\,\mu m$ lang und $0,1\,\mu m$ dick sind.

Dadurch kann eine besonders große Kraft durch die Verformung der Cantilever auf die Transducer erzeugt werden.

Die verformbaren Teile der Cantilever können beispielsweise anstatt einer rechteckigen Geometrie auch eine abweichende Geometrie aufweisen. Beispielsweise können die Cantilever eine V-förmige Geometrie aufweisen oder eine dreieckige Geometrie aufweisen. Insbesondere ist es auch möglich, dass der Cantilever kein geschlossener Körper ist, sondern beispielsweise Löcher oder Aussparungen aufweist.

Die Referenz- und Testcantilever können Si3N4 und/oder SiO2 und/oder Si3N4/SiO2 und/oder SiC und/oder Si und/oder Aluminiumoxid umfassen oder aus Si bestehen oder mindestens ein Polymer umfassen. Gleichermaßen können auch die Basen beziehungsweise die Gesamtbasis die den genannten Materialien umfassen. Die Basen und die Referenz- und Testcantilever können auch einteilig miteinander aus den genannten Materialien durch herkömmliche Herstellungsprozesse, wie sie bei der Bearbeitung von Wafern bekannt sind, hergestellt werden.

Durch die siliziumbasierten Referenz- und Testcantilever lassen sich aus der Halbleiterindustrie bekannte Herstellungsverfahren verwenden, sodass die Herstellung der vorgeschlagenen Sensoren in einem großen industriellen Maßstab ermöglicht wird. Polymere lassen sich ebenfalls in großem industriellem Maßstab herstellen und weisen den Vorteil auf, dass deren Materialeigenschaften in großem Umfang vorbestimmt werden können.

Die Transducer können identische intrinsische physikalische Eigenschaften aufweisen, wobei die Transducer dazu eingerichtet sind ihren elektrischen Eigenschaften, bevorzugt den elektrischen Widerstand oder einen anderen zum k-Wert proportionalen Wert, entsprechend den auf die Referenz- und Testcantilever wirkenden Kräfte anzupassen.

Der k-Wert, auch Gauge-faktor genannt, ist die Proportionalitätskonstante zwischen der Dehnung des Transducers und dessen Widerstandsänderung:

$$\frac{\Delta R}{R} = k \frac{\Delta L}{L},$$

wobei $\Delta R$ die Widerstandsänderung des Transducers ist, R der Widerstand des Transducers bei unverbogenem Cantilever, $\Delta L$ die Längenänderung des Transducers und $L$ die Länge des Transducers bei unverbogenem Cantilever ist. Insbesondere können auch alle anderen zum k-Wert beziehungsweise zum Widerstand proportionalen Messgrößen, wie beispielsweise die Leitfähigkeit, gemessen werden.

Identische intrinsische physikalische Eigenschaften umfassen hierbei diejenigen Eigenschaften, die für die Messeigenschaften des Transducers auf einem Cantilever verantwortlich sind. Dies betrifft insbesondere den Widerstand beziehungsweise die Leitfähigkeit des Transducers. Der Widerstand hängt insbesondere von der Geometrie des Transducers ab, sodass bei einer gleichförmigen Leitfähigkeit der verschiedenen Transducer dementsprechend die Geometrie der Transducer gleich sein muss. Insbesondere soll jeder Transducer gleich auf eine gleiche Krafteinwirkung beziehungsweise Verformung des Cantilevers reagieren.

Die intrinsischen physikalischen Eigenschaften werden insbesondere durch die Nanostruktur der Transducer bestimmt. Durch einen zuverlässigen Herstellungsprozess der Transducer kann somit gewährleistet werden, dass alle Transducer gleich auf eine Kraft reagieren, sodass Abweichungen der verschiedenen gemessenen Kräfte lediglich auf der äußeren Einwirkung auf die Cantilever beruhen und nicht von den intrinsischen physikalischen Eigenschaften abhängen.

Die Referenz- und Testcantilever, sowie die ersten und zweiten Referenz- und Testtransducer können spiegelsymmetrisch zueinander angeordnet sein.

Durch einen spiegelsymmetrischen Aufbau ist es möglich, dass äußere Störeinflüsse auf die Transducer reduziert werden, oder zumindest symmetrisch zueinander geführt werden. Dadurch kann die Messgenauigkeit und Störanfälligkeit verbessert werden.

Der Sensor kann Elektroden aufweisen, bevorzugt vier Elektroden aufweisen, die dazu eingerichtet sind, die Transducer elektrisch zu kontaktieren.

Eine Elektrode ist hierbei eine leitfähige Schicht, beispielsweise aus Gold, oder ein Draht beziehungsweise Kabel, welche von einem Anschlussende des Transducers eine elektrisch leitfähige Verbindung zu einem externen Gerät, wie beispielsweise einer Strom- oder Spannungsquelle beziehungsweise zu einem entsprechenden Messgerät herstellen kann. Prinzipiell kann jede leitfähige Verbindung zwischen dem Transducer und dem externen Gerät als Elektrode verstanden werden. Jedoch wird hier als Elektrode insbesondere der Teil der elektrischen Verbindung angesehen, die auf dem Sensor realisiert wird.

Typischerweise wird eine elektrische Verbindung vom Sensor zu einer externen Quelle oder einem Messgerät über einen elektrischen Anschluss realisiert. Hierbei wird ein elektrischer Anschlussstecker mit einem Kabel oder einem Draht auf ein sogenanntes Bondpad kontaktiert, beispielsweise in dem der Draht dort mit Ultraschall fest geschweißt wird. Von dem Bondpad führt anschließend eine elektrische Verbindung direkt zum Transducer. Die elektrische Isopotentialfläche zwischen Transducer und Bondpad wird im Folgenden Elektrode genannt.

Die Elektrode dient dazu die Transducer elektrisch zu kontaktieren und insbesondere die Möglichkeit zu schaffen die elektrischen Signale von dem Sensor an ein Messgerät zu führen.

Insbesondere können die Elektroden auf unterschiedlichen elektrischen Potenzialen liegen und über diese miteinander in Wechselwirkung treten. Um diese gegenseitige Beeinflussung der elektrischen Ströme und Spannungen in den Elektroden zu minimieren ist es daher vorteilhaft, wenn die Elektroden ebenfalls eine symmetrische Form aufweisen, sodass die jeweilige Störung zumindest gleichförmig auf das Gesamtsystem verteilt wird. Dies kann insbesondere dadurch realisiert werden, dass eine gerade Anzahl von Elektroden verwendet wird beziehungsweise dass bei vier Transducern lediglich vier Elektroden verwendet werden.

Durch die Auslegung der Elektrodengeometrie ist es daher möglich, dass der Grundsignalpegel, der sich an den Elektroden durch etwaige Potenzialdifferenzen ergibt, kleiner als 1,1V ist, sodass eine elektrische Verkapselung der Elektroden nicht notwendig ist. Unter elektrischer Verkapselung kann hierbei beispielsweise eine elektrische Isolation oder Abdeckung oder Abschirmung der Elektroden und der Bonddrähte verstanden werden. Dadurch kann der Herstellungsprozess vereinfacht werden die Messgenauigkeit wird verbessert.

Die Transducer können in einer Vollbrücke elektrisch verschaltet sein, wobei die Vollbrücke dazu eingerichtet ist, aufgrund der elektrischen Eigenschaften der Transducer, insbesondere bei einer asymmetrischen Änderung der elektrischen Eigenschaften der Transducer, eine Brückenquerspannung aufzubauen.

Eine Vollbrücke ist hierbei eine Messeinrichtung zur Messung von elektrischen Widerständen beziehungsweise von kleinen Widerstandsänderungen. Eine Vollbrücke ist auch bekannt unter den Bezeichnungen Wheatstone'sche Messbrücke oder H-Brücke oder symmetrische Vollbrücke oder thermisch-symmetrische Vollbrücke.

Im Grundzustand der Vollbrücke des Sensors ist die Brückenquerspannung idealerweise gleich null, da alle beteiligten Transducer eine gleiche Kraft detektieren. Dieser Grundzustand wird vorzugsweise bereits beim Herstellungsprozess eingestellt, sodass sich zwischen den Elektroden nur eine geringe Offsetspannung einstellt, die über einen messtechnischen Aufbau kompensiert werden kann.

Von diesem Grundzustand der Vollbrücke aus lassen sich dann bevorzugt asymmetrische Kraftänderungen detektieren. Wenn beispielsweise der erste Testtransducer des Testcantilevers auf eine Krafteinwirkung mit einer Änderung seiner elektrischen Eigenschaft respektive mit Änderung seines elektrischen Widerstandes reagiert, dann ist das Verhältnis der Widerstände in der Vollbrücke nicht mehr ausgeglichen, sodass sich eine Brückenquerspannung aufbaut. Die aufgebaute Brückenquerspannung kann schließlich mit einem Messgerät detektiert werden.

Über die Realisierung als Vollbrücke wird über die Referenztransducer des Referenzcantilevers quasi eine Kalibrierung der Testtransducers des Testcantilevers herbeigeführt.

Der Sensor kann einen Brückenquerspannungsdetektor umfassen, der dazu eingerichtet ist, die Brückenquerspannung der Vollbrücke zu detektieren, wobei durch die detektierte Brückenquerspannung auf das Vorkommen des durch die Rezeptorschicht selektiv aufgenommenen Analyten geschlossen wird, bevorzugt auf die Größe beziehungsweise Konzentration des Vorkommens geschlossen wird.

Ein Brückenquerspannungsdetektor kann insbesondere jeder Detektor sein, der in der Lage ist eine Spannung zu detektieren. Beispielsweise kann das ein Messwiderstand sein, oder ein Signalgeber oder ein Messgerät, welches die Spannung anzeigt oder eine andere Art von Detektor, der durch die Detektion einer Spannung ein Ausgabesignal erzeugt. Insbesondere kann der Brückenquerspannungsdetektor als A/D Wandler ausgebildet sein und die Brückenquerspannung in einen digitalen Wert umwandeln.

Idealerweise wird die Änderung der Brückenspannung als ratiometrische Änderung in Bezug auf eine definierte, sprich gemessene Versorgungsspannung ausgedrückt. Beispielsweise beeinflusst dann eine Drift in der Versorgungsspannung das Messsignal nicht.

Die ersten und zweiten Testtransducer können in je einer Vertiefung oder einer gemeinsamen Vertiefung des Testcantilevers angeordnet sein und die ersten und zweiten Referenztransducer können in je einer Vertiefung oder einer gemeinsamen Vertiefung des Referenzcantilevers angeordnet sein.

Eine Vertiefung kann hierbei beispielsweise einen rechteckigen Querschnitt in der Dickenrichtung des Cantilevers aufweisen und/oder einen rechteckigen Querschnitt in der Längsrichtung des Querschnitts aufweisen. Es kann aber auch sein, dass die Vertiefung beispielsweise einen teilweise elliptischen oder runden Querschnitt aufweist.

Eine Vertiefung kann hierbei einen Transducer aufnehmen, so dass der Transducer in der Vertiefung angeordnet wird. Insbesondere kann der Transducer die Vertiefung ausfüllen, so dass das Volumen der Vertiefung vollständig durch einen Transducer gefüllt wird. Es ist aber auch möglich, dass der Transducer lediglich einen Teil des Volumens füllt oder dass der Transducer lediglich auf einer Seite der Vertiefung angeordnet ist, beispielsweise auf der Bodenseite der Vertiefung. Die Bodenseite ist hierbei die Seite, die gegenüber der Oberfläche des Cantilevers abgesetzt ist.

Die Vertiefungen im Testcantilever und im Referenzcantilever können die Elastizität des Testcantilevers und des Referenzcantilevers lokal an der Stelle der Transducer erhöhen.

Dies liegt darin begründet, dass das Material der Cantilever ausgedünnt wird.

Die Testtransducer in den Vertiefungen des Testcantilevers können die Dehnbarkeit des Testcantilevers verringern und die Referenztransducer in den Vertiefungen des Referenzcantilevers können die Dehnbarkeit des Referenzcantilevers verringern.

Gewissermaßen sind die Transducer die Antagonisten der Vertiefungen und bewirken eine Verringerung der Dehnbarkeit. Aus diesem Grunde bewirken Transducer, die lediglich auf der Oberfläche eines Cantilevers aufgebracht sind, eine Verringerung der Dehnbarkeit und somit eine Verringerung der Messempfindlichkeit.

Die Transducer die in den Vertiefungen der Cantilever angeordnet sind detektieren jedoch die Dehnung eines Cantilevers, der eine große Dehnbarkeit aufweist, so dass eine hohe Messempfindlichkeit realisiert werden kann. Gleichzeitig ist der Transducer jedoch in einem Abstand zur neutralen Achse zu wählen, um überhaupt eine Messempfindlichkeit zu realisieren. Bevorzugt schneidet die neutrale Achse den Transducer entsprechend nicht, sondern liegt außerhalb der neutralen Achse.

Mathematisch lässt sich die gesteigerte Messempfindlichkeit wie folgt erklären. Für dünne und breite Cantilever ist die mechanische Spannung $\sigma$ des Cantilevers definiert durch

$$\sigma = \frac{My}{I},$$

wobei M das durch die z.B. chemische Wechselwirkung erzeugte Biegemoment ist und y der Abstand zur neutralen Achse des Cantilevers ist. Die neutrale Achse ist hierbei die Achse, entlang derer sich die im Material vorhandenen Spannungen gerade aufheben. Hierbei werden insbesondere alle Schichten des Cantilevers inklusive Aktivierungs- und Passivierungsschichten berücksichtigt. Für dünne und breite Cantilever ist das zweite Flächenmoment I beispielsweise gegeben durch

$$I = \frac{bh^3}{12},$$

wobei b die Breite des Cantilevers und h die Dicke des Cantilevers ist. Gleichzeitig ist die Dehnung des Cantilevers gegeben durch:

$$\epsilon = \frac{\sigma}{E},$$

wobei E der Young'sche Modulus ist. Es ergibt sich durch Einsetzen der obigen Gleichungen die Dehnung des Cantilevers in Abhängigkeit vom Abstand zur neutralen Achse:

$$\epsilon = \frac{12My}{Ebh^3}.$$

Indem der Abstand y des Transducers zur neutralen Achse bei konstantem Young'schen Modulus E verringert wird, sinkt zwar die Dehnung und somit auch die Empfindlichkeit des Cantilevers. Durch die geringere Versteifung des Cantilevers bewirkt durch die geringere Dicke wird die geringe Dehnung jedoch überkompensiert, so dass eine besonders hohe Empfindlichkeit des Cantilevers erreicht wird.

Mindestens eine Vertiefung kann tiefer als 5%, bevorzugt tiefer als 20%, besonders bevorzugt tiefer als 50% der Dicke des Cantilevers sein.

Je tiefer die Vertiefung, desto elastischer wird der Cantilever. Beispielsweise kann der Cantilever 500nm dick sein, so dass die Vertiefung tiefer als 25nm sein kann, bevorzugt tiefer als 100nm sein kann, besonders bevorzugt tiefer als 250nm sein kann.

Der Abstand mindestens eines Transdurcers zur neutralen Achse kann kleiner sein als 20%, bevorzugt kleiner als 10% besonders bevorzugt kleiner als 5% der Dicke des Cantilevers. Je kleiner der Abstand ist, desto größer ist die Messempfindlichkeit.

Die Höhe mindestens eines Transducers kann mindestens der Tiefe der Vertiefung entsprechen.

Insbesondere kann die Oberseite des Transducers mit der Oberfläche des Cantilevers abschließen. Es kann aber auch sein, dass die Oberseite des Transducers über der Oberfläche des Cantilevers liegt. In diesem Fall muss jedoch eine Kontaktierung über die Cantileverkante vorgenommen werden.

Durch die Wahl der Dicke des Transducers kann insbesondere die Steifigkeit des Cantilevers eingestellt werden.

Die Vertiefungen können auf der oberen und/oder der unteren Oberflächen des Cantilevers angeordnet sein.

Durch Vertiefungen auf der oberen und/oder der unteren Oberfläche können insbesondere verschiedene Biegemomente detektiert werden, so dass ein besonders großes Messsignal durch die Transducer erzeugt wird.

Im Gegensatz zum Stand der Technik wird somit nicht der Transducer ausgedünnt, um die Elastizität des Cantilevers trotz angeordnetem Transducer beizubehalten oder zu reduzieren, um ein möglichst großes Messsignal zu erhalten. Vielmehr wird der Cantilever hier ausgedünnt um den Abstand zur ursprünglichen neutralen Achse zu minimieren, um einen möglichst großes Messsignal zu erhalten.

Erfindungsgemäß sind die ersten und zweiten Transducer dazu eingerichtet, unterschiedliche Kraftkomponenten zu detektieren.

Je nach Orientierung der Transducer relativ zu den Achsen des Cantilevers, also insbesondere relativ zu der Längsachse, der Breitenachse und der Dickenachse des Cantilevers, wirken unterschiedliche Kräfte.

Insbesondere kann die Detektion unterschiedlicher Kraftkomponenten dazu genutzt werden, die Signalspreizung zwischen den ersten und zweiten Transducern eines Cantilevers zu vergrößern. Dadurch kann ein besonders großes Messsignal erzeugt werden.

Erfindungsgemäß ist der erste Transducer entlang der Längsachse des Cantilevers ausgerichtet und der zweite Transducer senkrecht zur Längsachse des Cantilevers ausgerichtet.

Der erste Transducer kann am Ort der maximalen Oberflächenspannung des Cantilevers angeordnet sein, und der zweite Transducer kann am Ort der minimalen Oberflächenspannung des Cantilevers angeordnet sein.

Der Ort der minimalen Oberflächenspannung kann insbesondere der Ort der negativsten Oberflächenspannung sein.

Dadurch kann ebenfalls ein besonders großes Messsignal erzeugt werden.

Durch einen Vergleich beider Messsignale zwischen Referenzcantilever und Testcantilever kann somit ein besonders großes Messsignal erzeugt werden.

Die oberen Oberflächen der Referenz- und Testcantilever können durch eine Aktivierungsschicht aktiviert sein, wobei die Aktivierungsschicht dazu eingerichtet ist, im Falle einer Krafteinwirkung auf den Referenz- und Testcantilever, eine im Vergleich zur nicht-aktivierten unteren Oberfläche des Referenz- und Testcantilever, eine größere Oberflächendehnung zur Verfügung zu stellen, und wobei die Aktivierungsschicht Gold oder andere chemisch inerte Materialien umfasst. Es ist jedoch auch möglich die untere Oberfläche zu aktivieren, wodurch sich die Beschreibung von oberer und unterer Oberfläche lediglich umdreht.

Eine Aktivierung der oberen Oberfläche kann bedeuten, dass durch Aufbringen einer Aktivierungsschicht eine Haftvermittlung für eine weitere Schicht zur Verfügung gestellt wird. Dies kann darin begründet sein das Basismaterial des Cantilevers beispielsweise keine Bindung mit der weiteren Schicht, insbesondere der Referenzschicht eingeht.

Insbesondere kann die Aktivierungsschicht Gold umfassen, oder ganz aus Gold bestehen.

Bevorzugt wird die komplette Oberfläche der Cantilever mit Gold bedeckt, da die Rezeptorschicht bevorzugt auf der Goldschicht aufbaut. Dementsprechend kann durch eine großflächige Schicht mit der Aktivierungsschicht auch eine größere Fläche mit der Rezeptorschicht belegt werden, sodass sich eine große Detektorfläche für den Analyten ergibt. Durch die große Detektorfläche für den Analyten wiederum ergibt sich eine besonders große Verformung der Cantilever, sodass eine empfindliche Detektion des Vorkommens des Analyten möglich ist.

Es ist aber auch möglich, dass lediglich der verformbare Teil der Cantilever mit Gold bedeckt ist, insbesondere bis zur Biegekante mit Gold bedeckt ist.

Indem die obere Oberfläche eine Aktivierungsschicht aufweist, ist insbesondere der Aufbau der Cantilever in der Höhe nicht homogen beziehungsweise asymmetrisch, sondern besteht aus Schichten. Dadurch kann die Elastizität des Cantilevers maßgeblich beeinflusst werden, sodass an der oberen Oberfläche bei einer Verformung der Cantilever eine größere Oberflächendehnung entsteht, die wiederum zu einem größeren Messsignal führt.

Die Beschichtung der Cantilever mit Gold kann aufgrund der guten Leitfähigkeit ebenfalls dazu verwendet werden Elektroden für die Transducer auszubilden. Aus diesem Grund kann auch der Abstand zwischen den Elektroden minimiert werden, da so möglichst wenig Fläche des Cantilevers nicht mit Gold belegt wird. Dementsprechend kann die Detektorfläche groß gewählt werden.

Die Aktivierungsschicht kann insbesondere auch aus einer Chrom-Gold-Legierung bestehen oder eine solche umfassen, da dadurch die mechanischen Eigenschaften des Cantilevers weniger beeinflusst werden. Insbesondere wird durch die Beimischung von Chrom eine Homogenität der Kristallite der Goldschicht erreicht, so dass eventuell störende Anisotropieeffekte durch das Kristallgitter einer hypothetischen kristallinen Schicht vermieden werden können.

Es ist aber auch möglich, dass die Aktivierungsschicht aus einer Titan-Gold-Legierung besteht oder eine solche umfasst.

Die unteren Oberflächen der Referenz- und Testcantilever können durch eine Passivierungsschicht passiviert sein, wobei die Passivierungsschicht dazu eingerichtet ist eine unspezifische Proteinadhäsion auf den Referenz- und Testcantilever zu minimieren, und wobei die Passivierungsschicht Trimethoxisilan und/oder eine Blocking-Substanz umfasst.

Im Unterschied zu einer Aktivierungsschicht ist eine Passivierungsschicht eine Schicht, die eine Wechselwirkung zwischen dem Cantilever und einem anderen Material minimieren oder unterbinden soll. Dies hat zur Folge, dass bei der Herstellung der Rezeptorschicht diese lediglich an der oberen Oberfläche des Cantilevers bindet und nicht an der unteren Oberfläche des Cantilevers bindet. Dadurch kann durch Bindung der Rezeptorschicht mit einem Analyten eine größere Oberflächenspannung an der oberen Oberfläche erreicht werden. Weiter wird dadurch die Asymmetrie des Schichtaufbaus verstärkt, was zu verbesserten Dehnungseigenschaften für die Signaldetektion führen kann.

Insbesondere eignen sich für die Passivierung der unteren Oberfläche die Materialien Trimethoxisilan, sowie sogenannte Blocking-Schichten. Durch diese Passivierungsschicht wird eine sogenannte unspezifische Proteinadhäsion minimiert. Die Proteinadhäsion ist eine Adhäsion eines Proteins an der Oberfläche. Eine unspezifische Adhäsion eines Proteins oder eines Stoffes im Allgemeinen, an den Cantilever kann zu Verzerrungen des Messergebnisses führen, da diese unspezifischen Stoffe ebenfalls mit den Cantilever in Wechselwirkung treten. In dem diese unspezifische Adhäsion unterbunden wird, vergrößert sich der relative Einfluss der gewollten spezifischen Adhäsion oder Wechselwirkung des Analyten mit dem Cantilever relativ zum Grundzustand des Cantilevers.

Es ist aber auch möglich, dass eine Passivierungsschicht auch den Analyten bindet, jedoch in einer Art und Weise, dass die daraus resultierende Oberflächenspannung an der Unterseite der Oberflächenspannung der Aktivierungsschicht an der Oberseite entgegengesetzt ist. Dadurch kann eine größere Verformung der Cantilever erreicht werden.

Die sogenannte Blocking-Schicht kann insbesondere auf den jeweils zu untersuchenden Analyten und bevorzugt auch die jeweilige Lösung, in welcher der Analyt vorliegt, angepasst werden, um gewissermaßen ein Messfenster für den Analyten zu definieren. Die Blocking-Schicht wird dabei im sogenannten Spottingprozess oder Waschprozess aufgebracht.

Beim Waschprozess schützt ein sogenannter "Sealer" beim Eintrocknen die Hydrathülle der Detektorproteine und macht diese somit lagerfähig, insbesondere ohne Kühlung bei Raumtemperatur lagefähig. Der Sealer ist löslich in einer Matrix eingebunden, so dass er löslich für eine Probenflüssigkeit wie Wasser ist. Zudem weist der Sealer eine gewisse Schichtdicke auf, so dass die Cantilever mechanisch stabilisiert werden, was den Schutz bei der Lagerung der Cantilever vergrößert. Ein Sealer kann beispielsweise Zucker enthalten. Die Zuckerkristalle sind hydrophil und schützen daher die Hydrathülle der Proteine. Somit ist eine sogenannte Rekonstituierung der Proteine, bei der die getrockneten Proteine in der Messflüssigkeit wieder aktiviert werden, möglich.

Beim Spotting der Rezeptorproteine werden sogenannte "Puffer" genutzt, um eine Rekonstituierung der Proteine in der Probenflüssigkeit zu ermöglichen. Auch hier wird durch ein Eintrocknen die Lagerfähigkeit der Sensoren erhöht.

Der Referenzcantilever und der Testcantilever können chemisch identisch aufgebaut sein.

Dadurch wird erreicht, dass das Messsignal, insbesondere bei einer differentiellen Messung der Brückenquerspannung, lediglich auf dem Einfluss des Analyten auf die Cantilever basiert und nicht durch weitere Eigenschaften der Cantilever hervorgerufen wird.

Insbesondere bezieht sich die chemische Identität darauf, dass die Cantilever insofern verändert und angepasst werden, als dass sie sich nur über ihre Bindungseigenschaften beziehungsweise Wechselwirkungseigenschaften an den zu messenden Analyten unterscheiden. Für alle weiteren Stoffe soll eine möglichst gleiche Wechselwirkung, beziehungsweise eine möglichst geringe Wechselwirkung erreicht werden.

Hierzu weisen der Referenzcantilever und der Testcantilever einen identischen Schichtaufbau auf, der sich lediglich darin unterscheidet, dass auf dem Testcantilever eine Rezeptorschicht aufgebracht ist und auf dem Referenzcantilever eine Referenzschicht. Insbesondere meint somit die chemische Identität, dass sich die beiden Cantilever nur in der Referenz- beziehungsweise Testschicht unterscheiden.

Insgesamt kann der oben beschriebene gesamte Schichtaufbau der Cantilever auch invertiert werden. Das bedeutet, dass die Referenz- und Rezeptorschichten anstatt auf der oberen Oberfläche auch auf der unteren Oberfläche der Cantilever aufgebracht werden können. Beispielsweise kann die Rezeptorschicht auch auf der Unterseite des Cantilevers angeordnet werden.

Damit sich der Cantilever verformt, sollte idealerweise jegliche chemische Anbindung an den Cantilever einseitig geschehen oder die Vorzeichen der chemischen Anbindung an der Oberseite und der Unterseite unterscheiden sich. Wenn der Analyt auf der Oberseite bindet, sollte auf der Unterseite des Cantilevers keine unspezifische Bindung geschehen, da sonst die Oberflächenspannung, die aus der chemischen Bindung des Analyten resultiert durch die unspezifische chemische Bindung auf der Unterseite des Cantilevers kompensiert werden kann.

Mit anderen Worten muss die chemische Anbindung auf der Oberseite und der Unterseite zumindest asymmetrisch sein, um eine Verformung zu erreichen. Eine stärkere Anbindung auf der Oberseite als auf der Unterseite oder eine stärkere Anbindung auf der Unterseite als auf der Oberseite führt entsprechend zu einer messbaren Verformung des Testcantilevers.

Die Referenz- und Testcantilever kann eine weitere Schicht aufweisen, die eine selbstorganisierende Monoschicht umfasst.

Eine selbstorganisierende Monoschicht kann insbesondere Unebenheiten auf der Goldoberfläche reduzieren, so dass eine gleichmäßige Beschichtung der Cantilever mit der Rezeptorbeziehungsweise Referenzschicht möglich ist. Durch die homogenen Oberflächeneigenschaften der Cantilever können sich so schließlich die Bindungseigenschaften der Rezeptorschicht und der Analyten verbessern.

Die Rezeptorschicht kann Antikörper für ein Antigen umfassen und die Referenzschicht kann einen auf den Antikörper der Referenzschicht ausgerichteten Antigen-spezifischen Isotypkontroll-Antikörper umfassen.

Antikörper sind beispielsweise Proteine die von Körperzellen als Reaktionsprodukt auf Antigene produziert werden. Antikörper werden typischerweise vom menschlichen Immunsystem dafür verwendet an die Antigene von Viren zu binden, sodass die Viren markiert und ein Ausbruch einer Virusinfektion durch das Immunsystem vermieden werden kann. Es gibt jedoch auch Antikörper, die an nicht immunologische Stoffe wie beispielsweise THC binden. Es kann insbesondere sein, dass ein Antikörper an verschiedene Antigene bindet, so dass die Spezifität des Antikörpers herabgesetzt ist.

Ein Isotypkontroll-Antikörper bindet im Gegensatz dazu genau nicht an das Antigen eines Virus, sodass bei gleichzeitigem Vorliegen einer Bindung des Antikörpers an das Antigen und eines nicht bindendes des Isotypkontroll-Antikörpers an das Antigen mit einer hohen Spezifität das Vorliegen eines bestimmten Virus beziehungsweise eines Antigens eines Virus geschlossen werden kann.

Der Antikörper eines Antigens kann Teil der Rezeptorschicht des Testcantilevers sein, während der Isotypkontroll-Antikörper des Antigens Teil der Referenzschicht sein kann. Das hat den Vorteil, dass eine Auslenkung des Testcantilevers gleichzeitig durch eine Nichtauslenkung des Referenzcantilevers bestätigt werden kann.

Die Referenz- und Rezeptorschicht der Cantilever können zudem für eine bessere Haftung der Antikörper das sogenannte Protein A aufweisen, welches kovalent an die selbstorganisierende Monoschicht bindet.

Die Schichten können in einem Dipping/Spotting- Prozess hergestellt werden, wobei das Spotting bevorzugt mittels kommerziell verfügbarer Maschinen durchgeführt werden kann. Hierbei werden Tröpfchen der jeweiligen Schicht auf den Cantilever abgelegt, so dass eine räumliche Begrenzung der Funktionalisierung erreicht wird, was insbesondere eine kostengünstige und unabhängige Beschichtung der Cantilever ermöglicht. Die sehr kleinen Tropfen werden durch eine geeignete Kontrolle der Umgebungsparameter, wie Temperatur, Luftfeuchte und Taupunkt, am trocknen gehindert. Die Unterseiten der Cantilever werden hierbei nicht aktiviert, sodass die verwendeten Antikörper bloß mit der oberen Oberfläche des Cantilevers in Kontakt kommen. Anschließend werden die Schichten getrocknet, so dass eine erhöhte oder erniedrigte Temperatur wenig oder bevorzugt kein Einfluss auf die Antikörper hat. Dies erlaubt eine lange Lagerfähigkeit, insbesondere in einem Inertgas. Die Proteinschichten werden insbesondere nach dem Aufbringen der Transducer aber vor der Vereinzelung der Sensoren oder Chips von dem Wafer aufgebracht.

Die Rezeptorschicht kann im Allgemeinen molekülspezifische Bindungskräfte bereitstellen und die Referenzschicht molekülspezifische keine Bindungskräfte bereitstellt. Dadurch ist es möglich eine bestimmte Molekülspezies nachzuweisen.

Die Rezeptorschicht kann Einzelstrang-DNA (ssDNA) und/oder andere DNA-Fragmente umfassen, die spezifisch an DNA-Fragmente in der Probe binden kann. Die Referenzschicht kann Einzelstrang-DNA und/oder andere DNA-Fragmente umfassen, die an keine chemische und/oder biochemische und/oder physikalische Spezies in der Probe bindet, aber in charakteristischen Parametern (z.B. Kettenlänge, chemischer Aufbau) mit der Rezeptorschicht übereinstimmt.

Die Rezeptorschicht kann Einzelstrang-RNA und/oder andere RNA-Fragmente umfassen, die spezifisch an RNA-Fragmente in der Probe binden kann. Die Referenzschicht kann Einzelstrang-RNA und/oder andere RNA-Fragmente umfassen, die an keine chemische und/oder biochemische und/oder physikalische Spezies in der Probe bindet, aber in charakteristischen Parameter (z.B. Kettenlänge, chemischer Aufbau) mit der Rezeptorschicht übereinstimmt. Dadurch ist es möglich eine bestimmte DNA oder RNA sowie deren Fragmente und/oder andere Oligonukleotide nachzuweisen.

Die Rezeptorschicht kann Antikörper und/oder andere und/oder weitere Proteine umfassen, die Zielproteine spezifisch binden können und die Referenzschicht kann entsprechend spezifische Isotypkontroll-Antikörper und/oder weitere Proteine umfassen, die an keine chemische und/oder biochemische und/oder physikalische Spezies in der Probe binden.

Die Rezeptorschicht kann scFv-Antikörper umfassen und die Referenzschicht kann scFv-Antikörper -spezifische Isotypkontroll-Antikörper umfassen. Ein scFv-Antikörper ist ein künstlich hergestelltes Antikörperfragment. Indem ein Antikörper in mehrere Fragmente zerlegt werden, kann die Reaktivität des Sensors auf eine geringe Probenkonzentration gesteigert werden. Beispielsweise kann der scFv-Antikörper in einer F(ab) oder einer F(ab')$_2$ oder einer F(ab') Konfiguration vorliegen.

Die Rezeptorschicht und/oder die Referenzschicht können Hydrogele umfassen.

Hydrogele sind molekulare Matrizen, die Wasser sehr gut binden können und die beim Kontakt mit Wasser stark anschwellen. Durch eine chemische Modifikation der Hydrogele, insbesondere der Matrix kann eine starke Reaktion des Hydrogels auf das Vorhandensein von Antikörpern bewerkstelligt werden, so dass die mechanische Verformung des Cantilevers vervielfacht wird. Insbesondere ist es so auch möglich eine pH-Wer-sensitive Messung des Analyten durchzuführen.

Kurze Beschreibung der Figuren

Ausführungsformen der Erfindung werden durch die nachfolgende Beschreibung der Figuren näher erläutert. Dabei zeigen:

| | |
|---|---|
| Figur 1 | eine schematische Darstellung eines nicht erfindungsgemäßen Sensors; |
| Figur 2A, B, C, D, E, F | schematische Darstellungen des Sensors mit verschiedenen Vertiefungen; |
| Figur 3A, B, C, D | schematische Darstellungen des Cantilevers mit Längsdehnungen und Querdehnungen; |
| Figur 4A, B | schematische Darstellungen eines erfindungsgemäßen Sensors und Konfigurationen der Transducer; |
| Figur 5A, B | weitere schematische Darstellung eines nicht erfindungsgemäßen Sensors mitsamt Sensorelektroden; und |
| Figur 6 | eine schematische Darstellung der Bindung von Antigenen an Antikörper. |

Detaillierte Beschreibung

Im Folgenden werden Ausführungsbeispiele anhand der Figuren beschrieben. Dabei werden gleiche, ähnliche oder gleichwirkende Elemente in den unterschiedlichen Figuren mit identischen Bezugszeichen versehen, und auf eine wiederholte Beschreibung dieser Elemente wird teilweise verzichtet, um Redundanzen zu vermeiden.

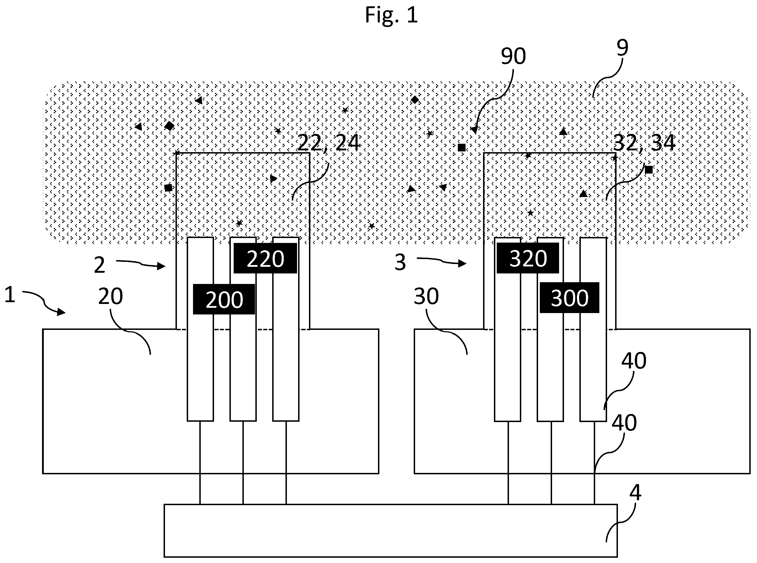

In Figur 1 ist schematisch ein nicht erfindungsgemäßer Sensors 1 zur Umwandlung chemischer und/oder biochemischer Information gezeigt. Der Sensor 1 umfasst einen Testcantilever 2, der eine Basis 20 sowie einen verformbaren Teil 22 aufweist. Auf dem verformbaren Teil 22 sind ein erster Testtransducer 200 und ein zweiter Testtransducer 220 angeordnet. Analog dazu weist der Sensor 1 auch einen Referenzcantilever 3 auf, der wiederum eine Basis 30 und einen verformbaren Teil 32 aufweist. Auf dem verformbaren Teil 32 sind ein erster Referenztransducer 300 und ein zweiter Referenztransducer 320 angeordnet.

Die Transducer 200, 220, 300, 320 sind jeweils über Elektroden 40 mit einer Elektronik 4 verbunden, die dazu in der Lage ist, die Messsignale der Transducer 200, 220, 300, 320 aufzuzeichnen oder weiterzuleiten, während die Elektronik 4 ebenfalls in der Lage ist, die Transducer 200, 220, 300, 320 mit Strom und/oder Spannung zu versorgen.

Der Sensor 1 hat die Aufgabe, das Vorkommen und bevorzugt die Menge des Vorkommens eines Analyten 90 in einer Probe 9 anzuzeigen. In der Figur 1 ist die Probe 9 eine Flüssigkeit, beispielsweise Lymphe oder eine verdünnte Lymphflüssigkeit. Es kann aber auch sein, dass die Probe 9 Speichel oder Blut oder eine andere Körperflüssigkeit ist. Es kann auch sein, dass die Probe 9 aus einer Gewebeentnahme stammt oder aus einem anderen entnommenen Stoff gewonnen und/oder synthetisiert wurde. Der Analyt 90 kann hierbei in der Probe gelöst sein, oder in einer ungelösten Art und Weise als Suspension oder Dispersion oder Emulsion vorliegen.

In jedem Fall soll mit dem Sensor 1 die Probe 9 mit Hinblick auf das Vorkommen und/oder eine Konzentration und/oder eine Menge des Analyten 90 untersucht werden. Zu diesem Zweck ist auf den Testcantilever 2 eine Rezeptorschicht 24 aufgebracht, mit der ein Analyt 90 in Wechselwirkung treten kann, beziehungsweise eine Rezeptorschicht 24 die den Analyten 90 adsorbieren oder absorbieren kann.

Durch die Wechselwirkung ändert sich die Oberflächenspannung des mit der Rezeptorschicht 24 belegten Abschnitts des verformbaren Teils 22 des Testcantilevers 2, was zu einer Verformung der des verformbaren Teils 22 des Testcantilevers 2 führt. Die ersten und zweiten Testtransducer 200, 220 registrieren daher eine Verformung des verformbaren Teils des Testcantilevers 2, was in der Elektronik 4 wiederum als Messsignal interpretiert wird.

Jedoch kann es bereits aufgrund der Wechselwirkung mit der Probenflüssigkeit 9 zur Registrierung einer Verformung durch die Testtransducer 200, 220 kommen, beispielsweise in dem lediglich die Oberflächenspannung der Flüssigkeit auf den verformbaren Teil 22 des Testcantilevers 2 wirkt und diesen verformt. Für eine solche Verformung ist demnach nicht das Vorhandensein eines Analyten 90 verantwortlich.

Um die Größe dieser Grundeinwirkung der Probe 9 auf den Testcantilever 2 festzustellen, wird gleichzeitig mit dem Testcantilever 2 der Referenzcantilever 3 mit der Probe 9 in Kontakt gebracht. Zu diesem Zweck weist der Referenzcantilever 3 eine Referenzschicht 34 auf, mit der ein Analyt 90 nicht in Wechselwirkung treten kann. Dadurch wird eine Differenzierung zum Messsignal des Testcantilevers 2 ermöglicht. Dementsprechend unterscheiden sich die Messsignale der Transducer 200, 220, 300, 320, sofern ein Analyt 90 in der Probe 9 vorkommt.

Der Testcantilever 2 und der Referenzcantilever 3 befinden sich jedoch an unterschiedlichen Positionen in der Probe 9, so dass unterschiedliche Umgebungsbedingungen, wie beispielsweise Temperaturschwankungen oder Konzentrationsgradienten etc., Einfluss auf die Messgenauigkeit nehmen.

Diese unterschiedlichen Umgebungsbedingungen können jedoch durch einen Vergleich der Messwerte der Transducer 200, 220, 300, 320 bereinigt werden. Demnach kann über den Sensor 1 das Vorkommen eines Analyten 90 in einer Probe 9 isoliert analysiert werden, indem durch eine Vielzahl an Messpunkten auf den Referenz- und Testcantilever 3, 2 der Einfluss von Wechselwirkungen, die nicht dem Analyten 90 zuzuordnen sind, reduziert und isoliert werden. Dies ermöglicht eine hohe Messgenauigkeit des Vorkommens des Analyten 90 in der Probe 9. Über die Größe des Unterschieds der Messsignale der Transducer 200, 220, 300, 320 des Testcantilevers 2 und des Referenzcantilevers 3 kann somit im einfachsten Fall direkt auf die Menge des Vorkommens des Analyten 90 in der Probe 9 geschlossen werden.

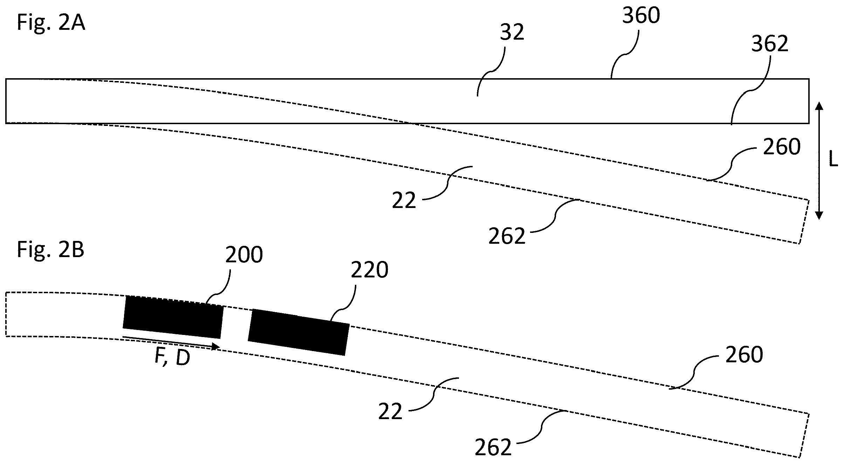

In Figur 2A ist der Vergleich der verformbaren Teile 32, 22 der Referenz-und Testcantilever 3, 2 bei einer Verformung und Längsdehnung gezeigt. Der verformbare Teil 32 des Referenzcantilevers 3 weist eine obere Oberfläche 360 und eine untere Oberfläche 362 auf. Ebenso weist der verformbare Teil 22 des Testcantilevers 2 eine obere Oberfläche 260 und eine untere Oberfläche 262 auf. Sofern ein Analyt 90 der Probe 9 mit dem Testcantilever 2, beziehungsweise mit der Rezeptorschicht 24 in Wechselwirkung tritt, findet eine Verformung des verformbaren Teils 22 vom ortsfesten Teil (der in die Basis des Testcantilevers übergeht) hin zum frei beweglichen Teil des verformbaren Teils 22 statt. Die gezeigte Auslenkung L ist hierbei gegeben durch die relative Auslenkung zwischen dem verformbaren Teil 32 des Referenzcantilevers 3 und dem verformbaren Teil 22 des Testcantilevers 2 aufgrund der Wechselwirkung mit dem Analyten 90.

Die Verformung des verformbaren Teils 22 des Testcantilevers 2 ist in Figur 2B exemplarisch für den Testcantilever 2 gezeigt. Die Beschreibung für den Referenzcantilever 3 erfolgt jedoch analog. Ursächlich hierfür ist, dass sich die obere Oberfläche 260 und die untere Oberfläche 262 des Testcantilevers 2 aufgrund der Wechselwirkung mit dem Analyten 90 unterschiedlich stark dehnen, so dass es zu einer Verformung des Testcantilever 2 kommt. Aufgrund der großen Dehnung D an der oberen Oberfläche 260, können die darauf aufgebrachten ersten und zweiten Testtransducer 200, 220 eine Dehnungskraft F registrieren. Die registrierte Dehnungskraft F kann hierbei durch die Testtransducer 200, 220 in ein elektronisches Signal umgewandelt werden beziehungsweise ein vorhandenes elektronisches Signal, beispielsweise eine anliegende Spannung, beeinflussen. Dies kann beispielsweise dadurch geschehen, dass die Testtransducer 200, 220 den Widerstand ändern, sofern sie eine Dehnungskraft F erfahren, die wiederum in einer Dehnung der Testtransducer 200, 220 resultiert.

Wie in Figur 2B gezeigt, können die ersten und zweiten Testtransducer 200, 220 in einer Vertiefung des Testcantilevers 2 angeordnet sein. Durch die Vertiefung wird die Elastizität des Testcantilevers 2 erhöht, wohingegen die ersten und zweiten Testtransducer 200, 220 die Steifigkeit des Testcantilevers 2 erhöhen. Durch die Kombination beider Effekte kann erreicht werden, dass die ersten und zweiten Testtransducer 200, 220 die Dehnung eines Testcantilevers mit einer hohen Elastizität vermessen, wodurch ein besonders großes Messsignal mit den Testtransducer, 200, erzeugt werden kann. Wenn die Testtransducer 200, 220 lediglich auf der Oberfläche des Cantilevers 2, 3 angeordnet wären und nicht in einer Vertiefung, dann wäre der Testcantilever 2 steifer, insbesondere im Bereich der jeweiligen Transducer, so dass ein schwächeres Messsignal erzeugt werden würde.

Die Vertiefungen können tiefer als 5%, bevorzugt tiefer als 20%, besonders bevorzugt tiefer als 50% der Dicke des Testcantilevers 2 sein. Im vorliegenden Fall in Figur 2B beträgt die Tiefe etwa 80% der Dicke des Cantilevers 2, 3.

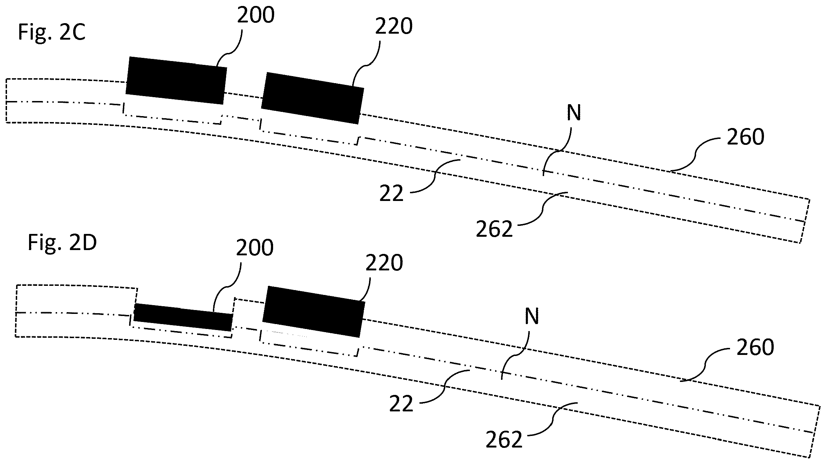

Zusätzlich entspricht die Höhe der ersten und zweiten Testtransducer 200, 220 der Tiefe der jeweiligen Vertiefung, so dass die obere Oberfläche der Testtransducer 200, 220 bündig mit der oberen Oberfläche 260 des Cantilevers 2 abschließt. Es ist aber auch möglich, dass die Testtransducer 200, 220 über die obere Oberfläche 260 hinausragen, wie in Figur 2C gezeigt, oder teilweise unter der oberen Oberfläche 260 liegen, wie in Figur 2D gezeigt, oder insgesamt unter der oberen Oberfläche 260 liegen (nicht gezeigt).

Zudem ist in den Figuren 2C und 2D die neutrale Achse N eingezeichnet, entlang derer im Grundzustand keine Materialspannung vorliegt, wobei insbesondere auch der Schichtaufbau berücksichtigt wird. Die neutrale Achse N kann beispielsweise über Computersimulationen des Schichtsystems mit der Geometrie des Cantilevers bestimmt werden.

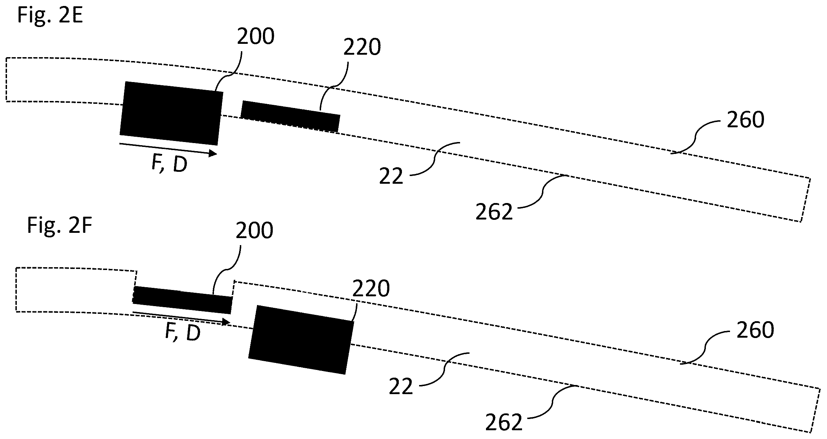

Die ersten und zweiten Testtransducer 200, 220 des Testcantilevers 2 können auch in Vertiefungen angeordnet sein, die auf der unteren Oberfläche des Testcantilevers 2 angeordnet sind, wie in Figur 2E gezeigt. Insbesondere ist es auch möglich, dass die Vertiefung des ersten Testtransducers 200 auf der oberen Oberfläche des Testcantilevers 2 angeordnet ist, während die Vertiefung des zweiten Testtransducers auf der unteren Oberfläche angeordnet ist (oder umgekehrt), wie in Figur 2F gezeigt. Insbesondere können die Testtransducer 200, 220 auch unterschiedliche Dicken aufweisen.

Über die Balkentheorie ist es beispielsweise möglich vorherzusagen, an welchen Stellen des verformbaren Teils 22 die Dehnung D am größten ist. Es ist möglich, dass an diesen Stellen die Testtransducer 200, 220 angeordnet werden, um ein optimales Signal-/Rauschverhältnis zu erzielen und um möglichst sensitiv auf die Dehnungen zu reagieren. Bei der genauen Positionierung der Testtransducer sollten jedoch auch andere Rahmenbedingungen berücksichtigt werden. Insbesondere können die Testtransducer 200, 220 auch an den Stellen des Testcantilevers 2 angeordnet werden, an der die Änderungen der Dehnung bei Kontakt mit einer Probe am größten sind.

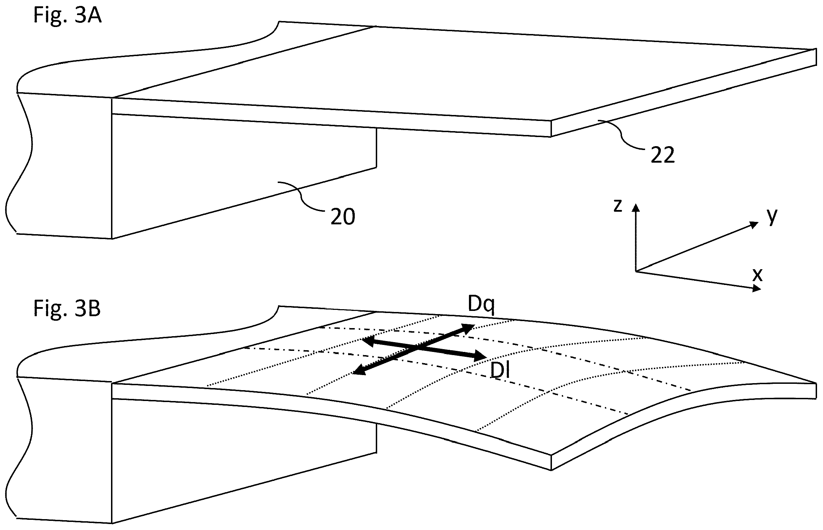

Insbesondere spielt die Ausrichtung der Testtransducer 200, 220 relativ zur Ausrichtung der Cantilever eine wichtige Rolle. In Figur 3A ist beispielsweise ein Testcantilever im Grundzustand gezeigt. Kommt der Testcantilever 2 in Kontakt mit dem Analyten 90, so ändert sich die Oberflächenspannung und es kommt zu einer Verformung des Materials, wie in Figur 3B gezeigt. In Figur 3B ist dargestellt, dass der Testcantilever eine Verformung senkrecht zur Basis 20, beziehungsweise zur Biegekante erfährt. Dies geht mit einer Längsausdehnung Dl der oberen Oberfläche einher. Gleichzeitig findet eine Verformung parallel zur Basis 20, beziehungsweise zur Biegekante statt, die mit einer Querausdehnung Dq der oberen Oberfläche einhergeht. Durch die Geometrie des Testcantilevers 2 kann festgelegt werden, entlang welcher Richtung eine größere Dehnung D bewirkt wird. Insbesondere können die Testtransducer 200, 220 entlang diesen Richtungen ausgerichtet werden, um ein besonders großes Messsignal zu erzeugen.

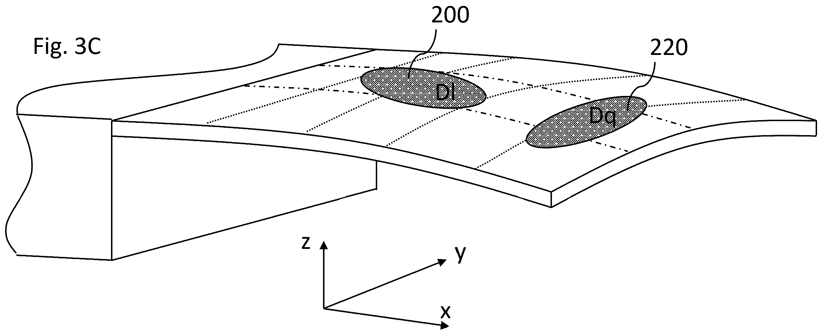

In Figur 3C ist exemplarisch gezeigt, dass ein erster Testtransducer 200 am Ort der größten Längsdehnung des Testcantilevers 2 angeordnet ist, während ein zweiter Testtransducer 220 am Ort der größten Querdehnung des Testcantilevers 2 angeordnet ist. Die Testtransducer 200, 220 sind hierbei elliptisch ausgebildet, sie können aber auch ein rechteckiges Profil aufweisen, wie in Figur 2 gezeigt. Insbesondere sind die beiden Testtransducer 200, 220 unterschiedlich bezüglich der Längsachse des Testcantilevers 2 ausgerichtet. Die Orientierung der Testtransducer 200, 220 kann sich beispielsweise nach der langen Achse der elliptischen Testtransducers 200, 220 richten. Dementsprechend kann ein isotropes Transducermaterial durch die geometrische Konfiguration eine Längs- oder eine Querdehnung oder auch einen Mischzustand detektieren.

Die Orientierung der Transducer ergibt sich aus einer Vorzugsrichtung der Transducer, in welcher jene die größtmögliche Sensitivität aufweisen. Diese ist typischerweise in der Richtung der größten Erstreckung der Transducer am höchsten. Bei einer Ausbildung der Transducer mit einer rechteckigen Grundfläche erstreckt sich die Vorzugsrichtung entsprechend entlang der längeren Seite des Rechtecks. Bei der hier angedeuteten elliptischen Grundfläche des Transducers erstreckt sich die Vorzugsrichtung entlang der Hauptachse.

Der erste Testtransducer 200 ist hierbei parallel zur Längsachse des Testcantilevers 2 ausgerichtet, während der zweite Testtransducer 220 senkrecht zur Längsachse des Testcantilevers 2 ausgerichtet ist. Insbesondere sind die beiden Testtransducer 200, 220 somit senkrecht zueinander orientiert und schließen einen Winkel von 90° ein.

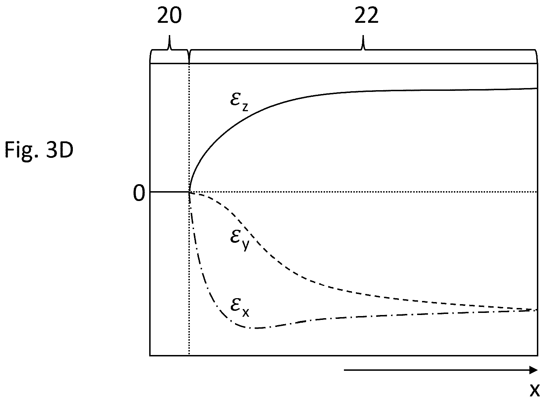

In Figur 3D ist schematisch der Verlauf der Dehnung des Testcantilevers in Figur 3C entlang der x-Achse gezeigt. Die Dehnung verschwindet hierbei entlang der Basis 20 und steigt ab der Biegekante im verformbaren Teil 22 betragsmäßig an. Insbesondere sind die Längsdehnung entlang der x-Achse und die Querdehnung entlang der y-Achse unterschiedlich groß.

Die obige Beschreibung der Figuren 2 und 3 gilt analog für die Funktionsweise des Referenzcantilevers 3 mit den ersten und zweiten Referenztransducer 300, 320.

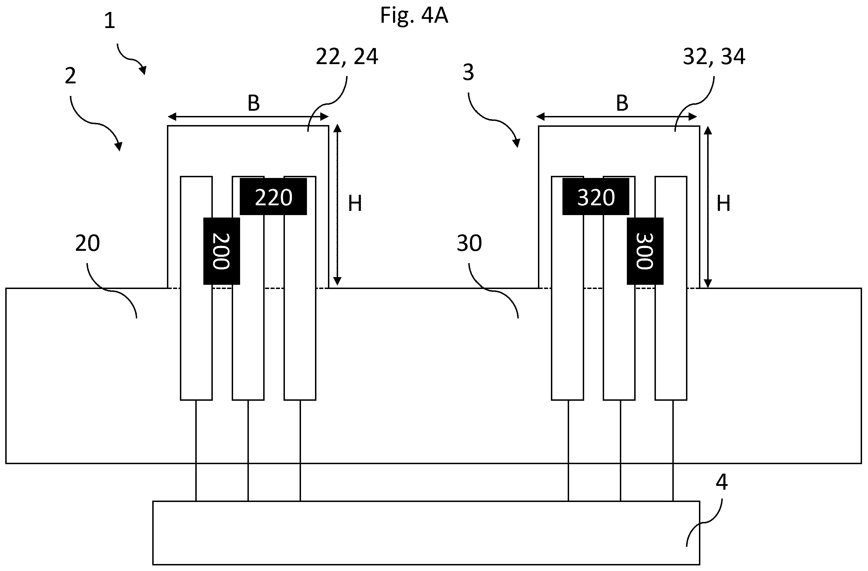

In Figur 4A ist eine erfindungsgemäße Ausführungsform des Sensors 1 gezeigt, bei der der Referenzcantilever 3 und der Testcantilever 2 identische geometrische Abmessungen aufweisen und spiegelsymmetrisch zueinander ausgestaltet sind. Insbesondere entsprechen die Höhe, Breite und Dicke des Referenzcantilevers 3 der Höhe, Breite und Dicke des Testcantilevers 2. Dadurch wird eine gleiche Dehnung D auf den oberen Oberflächen 260, 360 erzeugt. In dem die geometrischen Abmessungen der Cantilever 2, 3 identisch sind, wird dementsprechend auch eine gleiche Abhängigkeit des Messsignals von der Dehnung erwartet.

Beispielsweise ist die Breite B der Cantilever gleich der Höhe H der Cantilever 2, 3, wodurch eine besonders große Dehnung D an der oberen Oberfläche 260, 360 des Cantilevers 2, 3 ermöglicht wird. Beispielsweise sind dabei die Cantilever weniger als 150 µm breit, weniger als 150 µm lang und weniger als 1 µm dick, insbesondere 50 µm breit, 50 µm lang und 0,5 µm dick.

In der Ausführungsform des Sensors 1 in Figur 4A sind die Basen 30, 20 des Referenz-und Testcantilevers 3, 2 zudem auf derselben Gesamtbasis angeordnet, wodurch die Cantilever 2, 3 näher aneinander angeordnet werden können, um unterschiedliche Umgebungsbedingungen zu reduzieren.

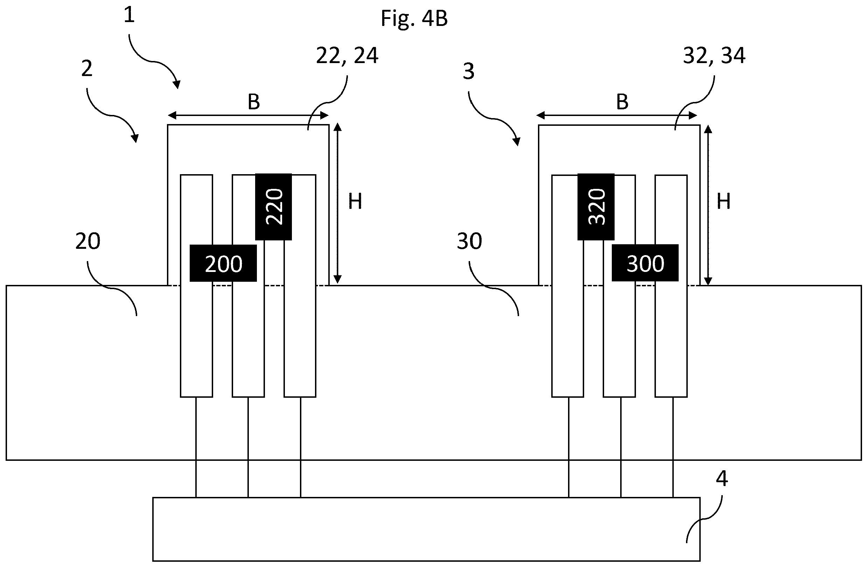

In Figur 4B ist eine weitere erfindungsgemäße Ausführungsform gezeigt, bei der die ersten Transducer 200, 300 senkrecht zur Längsachse der Cantilevers 2, 3 ausgerichtet sind und die zweiten Transducer 220, 320 parallel zur Längsachse der Cantilevers 2, 3 ausgerichtet sind. Zum Vergleich sind in Figur 4A die ersten Transducer 200, 300 parallel zur Längsachse der Cantilever 2, 3 ausgerichtet, während die zweiten Transducer senkrecht zur Längsachse der Cantilever 2, 3 ausgerichtet sind.

Indem die ersten Transducer 200, 300 beispielsweise eine Querausdehnung der Cantilever 2, 3 messen und die zweiten Transducer 220, 320 eine Längsausdehnung messen, ist der Unterschied in den Messsignalen der ersten Transducer 200, 300 beziehungsweise der zweiten Transducer 220, 320 lediglich auf die Wechselwirkung beziehungsweise Nicht-Wechselwirkung des Analyten mit den Cantilevern zurückzuführen.

Insbesondere können die gezeigten Transducer 200, 220, 300, 320 nicht nur auf der Oberfläche der Cantilever 2, 3 angeordnet und ausgerichtet werden, sondern auch in einer entsprechenden Vertiefung angeordnet sein.

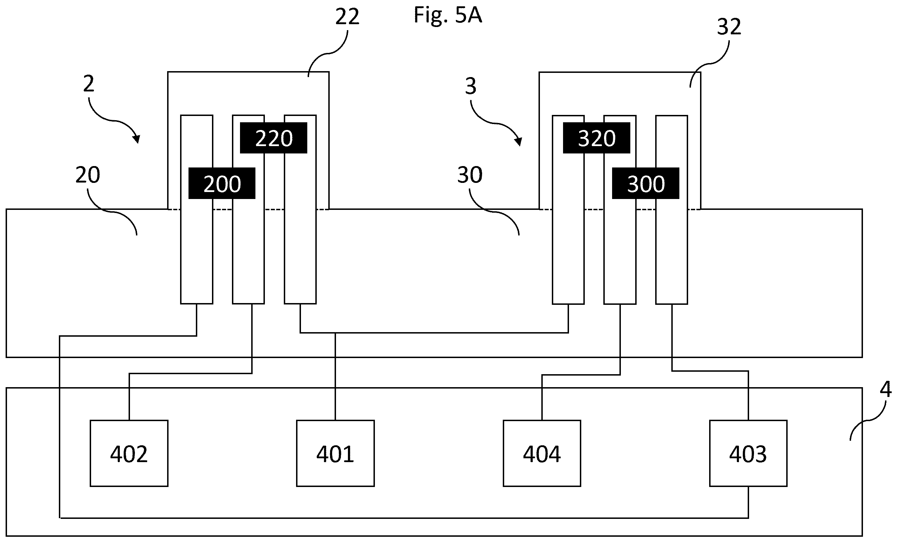

In Figur 5A ist eine weitere nicht erfindungsgemäße Ausführungsform des Sensors 1 gezeigt. Die Transducer 200, 220, 300, 320 sind über die Elektroden 401, 402, 403, 404 kontaktiert. Insbesondere ist der zweite Testtransducer 220 mit dem zweiten Referenztransducer 320 über die Elektrode 401 verbunden. Des Weiteren ist der erste Testtransducer 200 mit dem ersten Referenztransducer 300 über die Elektrode 403 verbunden. Der zweite Testtransducer 220 ist zudem mit dem ersten Testtransducer 200 über die Elektrode 402 verbunden, wohingegen der zweite Referenztransducer 320 mit dem ersten Referenztransducer 300 über die Elektrode 404 verbunden ist. Somit ergeben sich insgesamt vier Elektroden über die die Transducer 200, 220, 300, 320 miteinander elektrisch kontaktiert werden.

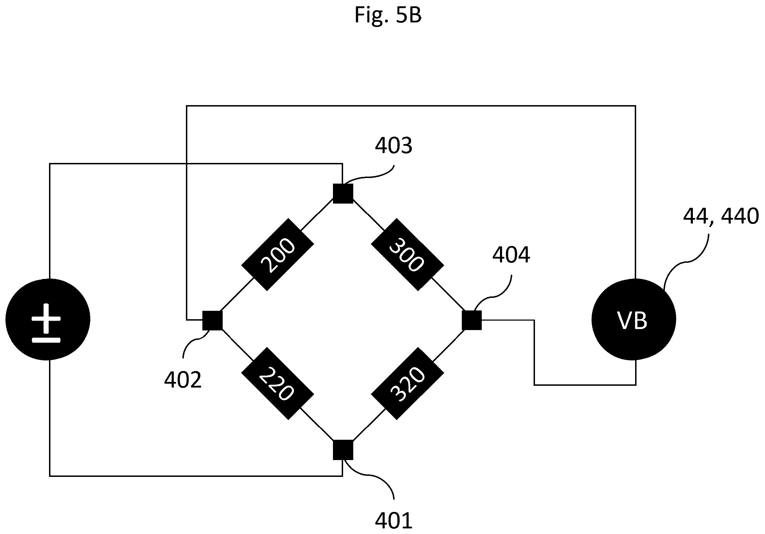

Die Transducer 200, 220, 300, 320 sind insbesondere in einer sogenannten Vollbrücke elektrisch verschaltet. Die Schaltung der Vollbrücke ist in Figur 5B gezeigt. Bei der Vollbrücke wird eine Gleichspannung oder Wechselspannung zwischen den Elektroden 403, 401 angelegt. Zwischen diesen Elektroden wirken die erste und zweiten Transducer als Spannungsteiler, aufgrund ihrer elektrischen Widerstände. Eine Vollbrücke in der gezeigten Form hat den Vorteil, dass zwischen den Elektroden 402, 404 keine Spannung aufgebaut wird, sofern das Verhältnis der Widerstände des ersten Testtransducers 200 zum zweiten Testtransducer 220 des Testcantilevers 2 gleich dem Verhältnis der Widerstände des ersten Referenztransducers 300 zum zweiten Referenztransducer 320 des Referenzcantilevers 3 ist. Es genügt also bereits die Abweichung eines Widerstandes, um die Widerstandsverhältnisse zu ändern, und um so eine Spannung zwischen den Elektroden 402, 404 aufzubauen.

Wenn der Referenzcantilever 3 und der Testcantilever 2 mit der Probe 9 und dem Analyten 90 in Wechselwirkung treten, so erfahren beide verformbaren Teile 22, 32 beispielsweise eine Änderung der Oberflächenspannung, die für den verformbaren Teil 22 des Testcantilevers 2 größer ist als für den verformbaren Teil 32 des Referenzcantilevers 3.

Indem sich die Widerstände der ersten und zweiten Transducer aufgrund beispielsweise der unterschiedlichen Ausrichtung unterschiedlich ändern, ergibt sich eine besonders große Änderung der Widerstandsverhältnisse aus der Verformung des verformbaren Teils 22 des Testcantilevers 2 aufgrund der Wechselwirkung mit dem Analyten 90 der Probe 9, die spezifisch mit der Referenzschicht 24 des Testcantilevers 2 wechselwirkt. Bei einer solchen Wechselwirkung wird dementsprechend eine Spannung zwischen den Elektroden 402, 404 aufgebaut, so dass eine Krafteinwirkung auf den ersten und zweiten Testtransducer 200, 220 relativ zum ersten und zweiten Referenztransducer 300, 320 als Brückenquerspannung VB angezeigt werden kann. Bevorzugt skaliert die Brückenquerspannung VB mit dem Vorkommen des Analyten 90 in der Probe 9, sodass eine quantitative Auswertung des Messsignals ermöglicht wird.

Ein Brückenquerspannungsdetektor 44 kann die Brückenquerspannung VB nach außen anzeigen oder weiterleiten, sodass für den Anwender des Sensors 1 sichtbar wird, dass eine Brückenquerspannung VB anliegt. Insbesondere kann ein solcher Brückenquerspannungsdetektor 44 auch durch einen AD-Wandler gegeben sein, wobei der AD-Wandler die Brückenquerspannung VB in ein Digitalsignal umwandelt, welches zur externen Messvorrichtung weitergeleitet werden kann. Es kann aber auch sein, dass der Detektor 44 die Signale an den Elektroden 402 beziehungsweise 404 isoliert voneinander detektiert, sodass eine Aussage über die jeweiligen Auslenkungen der verformbaren Teile 32, 22 getroffen werden kann.

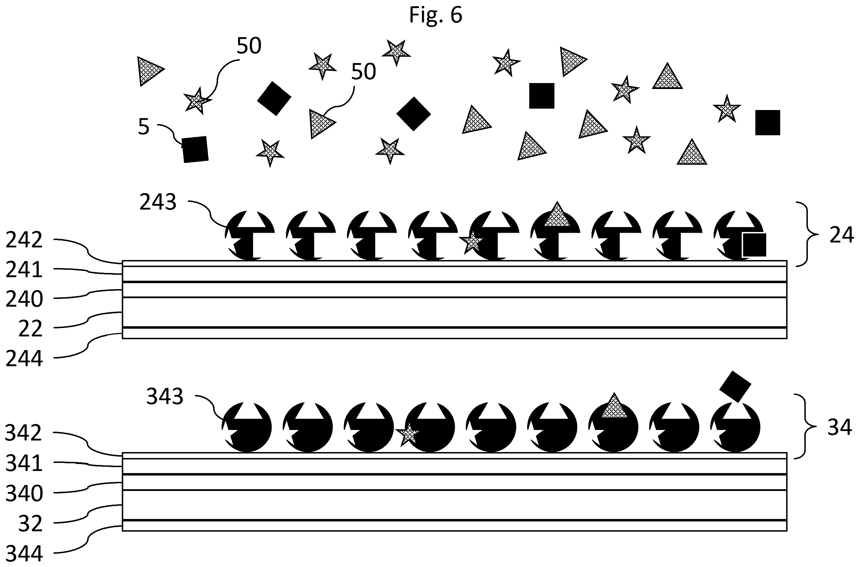

In Figur 6 ist schematisch der Aufbau der verschiedenen verformbaren Teile 22, 32 der Referenzbeziehungsweise Testcantilever 3, 2 gezeigt. Der Aufbau der Cantilever ist bis auf die Rezeptorschicht beziehungsweise die Referenzschicht identisch, so dass eine Wechselwirkung mit der Probe beziehungsweise dem umgebenden Medium als auch die mechanische Ausgestaltung des Cantilevers weitestgehend gleich ist.

Auf dem verformbaren Teil 32, 22 des Referenz- beziehungsweise Testcantilevers 3, 2 ist eine Aktivierungsschicht 34, 24 aufgebracht. Eine Aktivierungsschicht 240 ist dazu eingerichtet eine Haftvermittlung zwischen der Oberfläche des verformbaren Teils 32, 22 und einer weiteren Schicht 241, 341 zu realisieren. Des Weiteren hat die Aktivierungsschicht 240 die Aufgabe einen asymmetrischen Schichtaufbau des Cantilevers 3, 2 hervorzurufen, so dass es einen möglichst großen Unterschied in der Ausdehnung der oberen Oberfläche des Cantilevers und der unteren Oberfläche des Cantilevers gibt.

Auf die Goldschicht 240 kann sodann eine sogenannte selbstorganisierende Monoschicht 241 aufgebracht werden, welche die Oberflächenunebenheiten der Goldschicht ausgleichen kann und gleichzeitig für eine Haftvermittlung für eine weitere Schicht, nämlich die Referenzbeziehungsweise Rezeptorschichten 34, 24 bereitstellt.

Der Aufbau der Referenz- beziehungsweise Rezeptorschicht 34, 24 ist unterschiedlich. Beide Schichten basieren jedoch auf einer Schicht, die das sogenannte Protein A 242 umfassen kann, welches einerseits an die selbstorganisierende Monoschicht 241, 341 bindet, jedoch auf seiner Oberfläche auch Antikörper 243 beziehungsweise Isotypkontroll-Antikörper 343 aufweisen und binden kann.

Die Antikörper 243 sind Proteine die auf ein Antigen 5 reagieren, beziehungsweise mit diesem binden und somit beispielsweise im menschlichen Immunsystem Viruszellen markieren, sodass das Immunsystem den markierten Virus entsprechend vernichten kann, um beispielsweise einen Virusausbruch einzudämmen oder zu verhindern. Die Antikörper 243 sind weitestgehend spezifisch auf das Antigen 5, können jedoch auch mit anderen ähnlichen Antigenen 50 in Wechselwirkung treten. In der Figur 6 ist gezeigt, dass der Antikörper 243 gewissermaßen mit dem Antigen 5 und den ähnlichen Antigenen 50 in Wechselwirkung treten kann.

Im Gegensatz zum Antikörper 243 ist der Isotypkontroll-Antikörper 343 ein Protein welches bevorzugt ultrahochspezifisch nicht mit dem Antigen 5 in Wechselwirkung tritt. Dadurch kann eine Wechselwirkung mit einem spezifischen Antigen 5 quasi ausgeschlossen werden. Dies ist in der Figur 6 dadurch gezeigt, dass das Isotypkontroll-Antikörper 343 nur mit zwei ähnlichen Antigenen 50 in Wechselwirkung treten kann, jedoch nicht mit dem hier schematisch quadratisch dargestellten Antigen 5. Dadurch ist die relative Änderung der Oberflächenspannung der Cantilever 22, 32 lediglich auf das spezifische Antigen 5 zurückzuführen.

In dem der Testcantilever 2 einen Antikörper 243 aufweist und der Referenzcantilever 3 einen Isotypkontroll-Antikörper 343 aufweist wird sichergestellt, dass in der Probe 9 der Analyt 90, sofern der Analyt 90 ein Antigen 5 ist, nur mit dem Testcantilever 2 in Wechselwirkung treten kann.

Dadurch ist sichergestellt, dass die von dem Analyten 90 hervorgerufene relative Verformung des Testcantilevers 2 im Vergleich zur Verformung des Referenzcantilevers 3 nur auf der Anwesenheit des Analyten 90, beziehungsweise des Antigen 5 basiert. Demnach ist mit diesem Sensor 1 möglich ein Antigen 5 sicher und schnell zu detektieren.

Im Gegensatz zur oberen Oberfläche der Cantilever 2, 3 ist die untere Oberfläche der Cantilever passiviert. Eine solche Passivierung 244 und 344kann dazu führen, dass eine Wechselwirkung, beziehungsweise Bindung, beziehungsweise Absorption oder Absorption eines Analyten 90 der Probe 9 in oder auf den Cantilever vermieden wird. Insbesondere trägt eine solche Passivierungsschicht jedoch auch dazu bei, die Asymmetrie des Schichtaufbaus zu vergrößern, um einen möglichst großen Dehnungseffekt an der oberen Oberfläche des Cantilevers 3, 2 hervorzurufen. Insbesondere kann die Passivierungsschicht Trimethoxisilan und/oder eine Blocking-Substanz umfassen.

Bezugszeichenliste

| 1 | Sensor |
|---|---|
| 10 | Biegekante |
| 2 | Testcantilever |
| 20 | Basis |
| 200 | erster Testtransducer |
| 22 | verformbarer Teil |

| 220 | zweiter Testtransducer |
|---|---|
| 24 | Rezeptorschicht |
| 240 | Aktivierungsschicht |
| 241 | selbstorganisierende Monoschicht |
| 242 | Protein A |
| 243 | Antikörper |
| 244 | Passivierungsschicht |
| 26 | Oberfläche |
| 260 | obere Oberfläche |
| 262 | untere Oberfläche |
| 3 | Referenzcantilever |
| 30 | Basis |
| 300 | erster Referenztransducer |
| 32 | verformbarer Teil |
| 320 | zweiter Referenztransducer |
| 34 | Referenzschicht |
| 340 | Aktivierungsschicht |
| 341 | selbstorganisierende Monoschicht |
| 342 | Protein A |
| 343 | Isotypkontroll-Antikörper |
| 344 | Passivierungsschicht |
| 36 | Oberfläche |
| 360 | obere Oberfläche |
| 362 | untere Oberfläche |
| 4 | Elektronik |
| 40 | Elektrode |
| 400, 401, 402, 403 | Elektroden |
| 42 | Brückenquerspannungsdetektor |
| 44 | AD-Wandler |
| 440 | AD-Wandlerlogik |
| 5 | Antigen |
| 50 | ähnliches Antigen |
| 9 | Probe |
| 90 | Analyt |
| F | Kraft |
| L | Auslenkung |
| D | Dehnung |
| AT | Abstand zwischen aktiven und passiven Transducer |
| AE | Abstand zwischen Elektroden |
| S | Symmetrieachse |
| VB | Brückenquerspannung |
| N | Neutrale Achse |

**Patentansprüche**

1. Sensor (1) zur Umwandlung chemischer und/oder biochemischer Information eines Analyten (90) in einer Probe (9) in ein elektrisches Signal, umfassend

einen Testcantilever (2), der eine Basis (20) und einen verformbaren Teil (22) aufweist, wobei mindestens auf dem verformbaren Teil eine Rezeptorschicht (24) zur selektiven Aufnahme des Analyten (90) aufgebracht ist, wobei auf dem Testcantilever (2) ein erster und zweiter Testtransducer (200, 220) angeordnet ist,
einen Referenzcantilever (3), der eine Basis (30) und einen verformbaren Teil (32) aufweist, wobei mindestens auf dem verformbaren Teil (32) eine Referenzschicht (34) zur selektiven Nichtaufnahme des Analyten (90) aufgebracht ist, wobei auf dem Referenzcantilever (3) ein erster und zweiter Referenztransducer (320) angeordnet ist,
wobei die Transducer (200, 220, 300, 320) dazu ausgebildet und eingerichtet sind, ein dem Vorkommen und/oder der Konzentration und/oder der Menge des Analyten (90) in der Probe (9) entsprechendes elektrisches Signal auszugeben,

wobei durch die selektive Nichtaufnahme des Analyten (90) durch die Referenzschicht (34), die Wechselwirkung des Referenzcantilevers (3) mit der Probe (9) mit Analyten (90), der Wechselwirkung des Testcantilevers (2) mit der Probe (9) ohne Analyten (90) entspricht,

**dadurch gekennzeichnet, dass**

die ersten und zweiten Testtransducer (200, 220) auf dem verformbaren Teil (22) des Testcantilevers (2) angeordnet sind und die ersten und zweiten Referenztransducer (300, 320) auf dem verformbaren Teil (32) des Referenzcantilevers (3) angeordnet sind,

wobei die ersten und zweiten Transducer (200, 220, 300, 320) desselben Cantilevers (2, 3) dazu eingerichtet sind, unterschiedliche Kraftkomponenten zu detektieren,

wobei der erste Transducer (200, 300) entlang der Längsachse des Cantilevers (2, 3) ausgerichtet ist und der zweite Transducer (220, 320) senkrecht zur Längsachse des Cantilevers (2, 3) ausgerichtet ist.

2. Sensor (1) nach Anspruch 1, **dadurch gekennzeichnet, dass** die zu detektierende Kraft auf der Biegesteifigkeit der Referenz- und Testcantilever (3, 2) beruht.

3. Sensor (1) nach Anspruch 1 oder 2, **dadurch gekennzeichnet, dass** durch Vergleich der durch die Transducer (320, 300, 220, 200) detektierten Kräfte auf eine durch die selektive Aufnahme des Analyten (90) verursachten Einwirkung auf den Testcantilever (2) und somit auf dessen Vorkommen, geschlossen wird geschlossen wird.

4. Sensor (1) nach einem der vorherigen Ansprüche, **dadurch gekennzeichnet, dass** die verformbaren Teile (32, 22) der Referenz- und Testcantilever (3, 2) identische geometrische Abmessungen aufweisen wobei bevorzugt die Breite des verformbaren Teils der Referenz-und Testcantilever der Länge des verformbaren Teils der Referenz- und Testcantilever entspricht.

5. Sensor (1) nach einem der vorherigen Ansprüche, **dadurch gekennzeichnet, dass** die Referenz- und Testcantilever (3, 2) Si und/oder Aluminiumoxid und/oder mindestens ein Polymer umfassen.

6. Sensor (1) nach einem der vorherigen Ansprüche, **dadurch gekennzeichnet, dass** die Transducer (320, 300, 220, 200) identische intrinsische physikalische Eigenschaften aufweisen, wobei die Transducer (320, 300, 220, 200) dazu eingerichtet sind ihre elektrischen Eigenschaften entsprechend den auf die Referenz- und Testcantilever (3, 2) wirkenden Kräfte anzupassen.

7. Sensor (1) nach einem der vorherigen Ansprüche, **dadurch gekennzeichnet, dass** die Referenz- und Testcantilever (3, 2), sowie die aktiven und passiven Referenz- und Testtransducer (320, 300, 220, 200) spiegelsymmetrisch zueinander angeordnet sind.

8. Sensor (1) nach einem der vorherigen Ansprüche, **dadurch gekennzeichnet, dass** die ersten und zweiten Testtransducer (200, 220) in je einer Vertiefung oder einer gemeinsamen Vertiefung des Testcantilevers (2) angeordnet sind und die ersten und zweiten Referenztransducer (300, 320) in je einer Vertiefung oder einer gemeinsamen Vertiefung des Referenzcantilevers (3) angeordnet sind.

9. Sensor (1) nach Anspruch 8, **dadurch gekennzeichnet, dass** die Vertiefungen auf der oberen und/oder der unteren Oberflächen (260, 262, 360, 362) des Cantilevers (2, 3) angeordnet sind.

10. Sensor (1) nach Anspruch 9, **dadurch gekennzeichnet, dass** die mindestens eine Vertiefung tiefer ist als 5%der Dicke des Cantilevers (2, 3) ist, und der Abstand mindestens eines Transdurcers zur neutralen Achse des Cantilevers kleiner als 20% der Dicke des Cantilevers (2, 3) ist

11. Sensor (1) nach Anspruch 9 oder 10, **dadurch gekennzeichnet, dass** die Höhe mindestens eines Transducers (200, 220, 300, 320) mindestens der Tiefe der Vertiefung entspricht.

12. Sensor (1) nach Anspruch 11, **dadurch gekennzeichnet, dass**der erste und der zweite Transducer (200, 220, 300, 320) am Ort der maximalen und der minimalen Oberflächenspannung des Cantilevers (2, 3) angeordnet sind.

13. Sensor (1) nach einem der vorherigen Ansprüche, **dadurch gekennzeichnet, dass** die oberen Oberflächen (360, 260) der Referenz- und Testcantilever (3, 2) durch eine Aktivierungsschicht (340, 240) aktiviert sind,

wobei die Aktivierungsschicht (340, 240) dazu eingerichtet ist, eine im Vergleich zur nicht-aktivierten unteren Oberfläche (362, 262) des Referenz- und Testcantilever (3, 2), größere Oberflächenspannung zur Verfügung zu stellen, und

wobei die Aktivierungsschicht (340, 240) Gold umfasst.

**14.** Sensor (1) nach einem der vorherigen Ansprüche, **dadurch gekennzeichnet, dass** die oberen oder unteren Oberflächen (362, 262) der Referenz- und Testcantilever (3, 2) durch eine Passivierungsschicht (344, 244) passiviert sind,

wobei die Passivierungsschicht (344, 244) dazu eingerichtet ist eine unspezifische Proteinadhäsion auf dem Referenz- und Testcantilever (3, 2) zu minimieren, und

wobei die Passivierungsschicht (344, 244) Trimethoxisilan und/oder eine Blocking-Substanz umfasst.

**15.** Sensor (1) nach einem der vorherigen Ansprüche, **dadurch gekennzeichnet, dass** die Referenz- und Testcantilever (3, 2) eine weitere Schicht (341, 241) aufweisen, die eine selbstorganisierende Monoschicht umfasst.

## Claims

**1.** Sensor (1) for converting chemical and/or biochemical information of an analyte (90) in a sample (9) into an electrical signal, comprising

a test cantilever (2) which has a base (20) and a deformable part (22), wherein a receptor layer (24) for selectively receiving the analyte (90) is applied at least on the deformable part, wherein a first and second test transducer (200, 220) are arranged on the test cantilever (2),
a reference cantilever (3) which has a base (30) and a deformable part (32), wherein a reference layer (34) for selectively not-receiving the analyte (90) is applied at least on the deformable part (32), wherein a first and second reference transducer (320) are arranged on the reference cantilever (3),
wherein the transducers (200, 220, 300, 320) are designed and configured to output an electrical signal corresponding to the occurrence and/or the concentration and/or the amount of the analyte (90) in the sample (9),
wherein, as a result of the selective not-receiving of the analyte (90) by the reference layer (34), the interaction of the reference cantilever (3) with the sample (9) with analyte (90) corresponds to the interaction of the test cantilever (2) with the sample (9) without analyte (90),
**characterized in that**
the first and second test transducers (200, 220) are arranged on the deformable part (22) of the test cantilever (2) and the first and second reference transducers (300, 320) are arranged on the deformable part (32) of the reference cantilever (3),
wherein the first and second transducers (200, 220, 300, 320) of the same cantilever (2, 3) are configured to detect different force components,
wherein the first transducer (200, 300) is aligned along the longitudinal axis of the cantilever (2, 3) and the second transducer (220, 320) is aligned perpendicularly to the longitudinal axis of the cantilever (2, 3).

**2.** Sensor (1) according to claim 1, **characterized in that** the force to be detected is based on the bending stiffness of the reference and test cantilevers (3, 2).

**3.** Sensor (1) according to claim 1 or 2, **characterized in that,** by comparing the forces detected by the transducers (320, 300, 220, 200), an effect on the test cantilever (2) caused by the selective receiving of the analyte (90) and thus the occurrence thereof is concluded.

**4.** Sensor (1) according to one of the preceding claims, **characterized in that** the deformable parts (32, 22) of the reference and test cantilevers (3, 2) have identical geometric dimensions, wherein preferably the width of the deformable part of the reference and test cantilevers corresponds to the length of the deformable part of the reference and test cantilevers.

**5.** Sensor (1) according to one of the preceding claims, **characterized in that** the reference and test cantilevers (3, 2) comprise Si and/or aluminium oxide and/or at least one polymer.

6. Sensor (1) according to one of the preceding claims, **characterized in that** the transducers (320, 300, 220, 200) have identical intrinsic physical properties, wherein the transducers (320, 300, 220, 200) are configured to adapt their electrical properties corresponding to the forces acting on the reference and test cantilevers (3, 2).

7. Sensor (1) according to one of the preceding claims, **characterized in that** the reference and test cantilevers (3, 2) and the active and passive reference and test transducers (320, 300, 220, 200) are arranged mirror-symmetrically with respect to one another.

8. Sensor (1) according to one of the preceding claims, **characterized in that** the first and second test transducers (200, 220) are each arranged in a recess or a common recess of the test cantilever (2) and the first and second reference transducers (300, 320) are each arranged in a recess or a common recess of the reference cantilever (3).

9. Sensor (1) according to claim 8, **characterized in that**
the recesses are arranged on the upper and/or the lower surfaces (260, 262, 360, 362) of the cantilever (2, 3).

10. Sensor (1) according to claim 9, **characterized in that** the at least one recess is deeper than 5% of the thickness of the cantilever (2, 3), and
the distance of at least one transducer from the neutral axis of the cantilever is less than 20% of the thickness of the cantilever (2, 3).

11. Sensor (1) according to claim 9 or 10, **characterized in that** the height of at least one transducer (200, 220, 300, 320) corresponds at least to the depth of the recess.

12. Sensor (1) according to claim 11, **characterized in that** the first and the second transducer (200, 220, 300, 320) are arranged at the location of the maximum and the minimum surface tension of the cantilever (2, 3).

13. Sensor (1) according to one of the preceding claims, **characterized in that** the upper surfaces (360, 260) of the reference and test cantilevers (3, 2) are activated by an activation layer (340, 240),

wherein the activation layer (340, 240) is configured to provide a greater surface tension compared to the non-activated lower surface (362, 262) of the reference and test cantilevers (3, 2), and
wherein the activation layer (340, 240) comprises gold.

14. Sensor (1) according to one of the preceding claims, **characterized in that** the upper or lower surfaces (362, 262) of the reference and test cantilevers (3, 2) are passivated by a passivation layer (344, 244),

wherein the passivation layer (344, 244) is configured to minimize non-specific protein adhesion on the reference and test cantilevers (3, 2), and
wherein the passivation layer (344, 244) comprises trimethoxysilane and/or a blocking substance.

15. Sensor (1) according to one of the preceding claims, **characterized in that** the reference and test cantilevers (3, 2) have a further layer (341, 241) which comprises a self-organising monolayer.

**Revendications**

1. Capteur (1) pour la conversion d'informations chimiques et/ou biochimiques d'un analyte (90) dans un échantillon (9) en un signal électrique, comprenant

un porte-à-faux de test (2), qui présente une base (20) et une partie déformable (22), une couche de récepteur (24) pour l'absorption sélective de l'analyte (90) étant appliquée au moins sur la partie déformable, un premier et un deuxième transducteur de test (200, 220) étant disposés sur le porte-à-faux de test (2),
un porte-à-faux de référence (3), qui présente une base (30) et une partie déformable (32), une couche de référence (34) pour la non-absorption sélective de l'analyte (90) étant appliquée au moins sur la partie déformable (32), un premier et un deuxième transducteur de référence (320) étant disposés sur le porte-à-faux de référence (3),
les transducteurs (200, 220, 300, 320) étant réalisés et conçus pour émettre un signal électrique correspondant à la présence et/ou à la concentration et/ou à la quantité de l'analyte (90) dans l'échantillon (9),

la non-absorption sélective de l'analyte (90) par la couche de référence (34), l'interaction du porte-à-faux de référence (3) avec l'échantillon (9) avec l'analyte (90), correspondant à l'interaction du porte-à-faux de test (2) avec l'échantillon (9) sans analyte (90),

**caractérisé en ce que**

les premier et deuxième transducteurs de test (200, 220) sont disposés sur la partie déformable (22) du porte-à-faux de test (2) et les premier et deuxième transducteurs de référence (300, 320) sont disposés sur la partie déformable (32) du porte-à-faux de référence (3),

les premier et deuxième transducteurs (200, 220, 300, 320) du même porte-à-faux (2, 3) étant conçus pour détecter différentes composantes de force,

le premier transducteur (200, 300) étant orienté le long de l'axe longitudinal du porte-à-faux (2, 3) et le deuxième transducteur (220, 320) étant orienté perpendiculairement à l'axe longitudinal du porte-à-faux (2, 3).

2. Capteur (1) selon la revendication 1, **caractérisé en ce que** force à détecter repose sur la rigidité en flexion des porte-à-faux de référence et de test (3, 2).

3. Capteur (1) selon la revendication 1 ou 2, **caractérisé en ce qu'**une comparaison des forces détectées par les transducteurs (320, 300, 220, 200) permet de conclure à une action provoquée par l'absorption sélective de l'analyte (90) sur le porte-à-faux de test (2) et donc à sa présence.

4. Capteur (1) selon l'une quelconque des revendications précédentes, **caractérisé en ce que** les parties déformables (32, 22) des porte-à-faux de référence et de test (3, 2) présentent des dimensions géométriques identiques, la largeur de la partie déformable des porte-à-faux de référence et de test correspondant de préférence à la longueur de la partie déformable des porte-à-faux de référence et de test.

5. Capteur (1) selon l'une quelconque des revendications précédentes, **caractérisé en ce que** les porte-à-faux de référence et de test (3, 2) comprennent du Si et/ou de l'oxyde d'aluminium et/ou au moins un polymère.

6. Capteur (1) selon l'une quelconque des revendications précédentes, **caractérisé en ce que** les transducteurs (320, 300, 220, 200) présentent des propriétés physiques intrinsèques identiques, les transducteurs (320, 300, 220, 200) étant conçus pour adapter leurs propriétés électriques en fonction des forces agissant sur les porte-à-faux de référence et de test (3, 2).

7. Capteur (1) selon l'une quelconque des revendications précédentes, **caractérisé en ce que** les porte-à-faux de référence et de test (3, 2) ainsi que les transducteurs de référence et de test actifs et passifs (320, 300, 220, 200) sont disposés en symétrie spéculaire les uns par rapport aux autres.

8. Capteur (1) selon l'une quelconque des revendications précédentes, **caractérisé en ce que** les premier et deuxième transducteurs de test (200, 220) sont disposés dans respectivement un creux ou un creux commun du porte-à-faux de test (2) et les premier et deuxième transducteurs de référence (300, 320) sont disposés dans respectivement un creux ou un creux commun du porte-à-faux de référence (3).

9. Capteur (1) selon la revendication 8, **caractérisé en ce que** les creux sont disposés sur la surface supérieure et/ou la surface inférieure (260, 262, 360, 362) du porte-à-faux (2, 3).

10. Capteur (1) selon la revendication 9, **caractérisé en ce que** l'au moins un creux est plus profond que 5 % de l'épaisseur du porte-à-faux (2, 3), et
la distance d'au moins un transducteur à l'axe neutre du porte-à-faux est inférieure à 20 % de l'épaisseur du porte-à-faux (2, 3).

11. Capteur (1) selon la revendication 9 ou 10, **caractérisé en ce que** la hauteur d'au moins un transducteur (200, 220, 300, 320) correspond au moins à la profondeur du creux.

12. Capteur (1) selon la revendication 11, **caractérisé en ce que** les premier et deuxième transducteurs (200, 220, 300, 320) sont disposés à l'emplacement de la tension superficielle maximale et minimale du porte-à-faux (2, 3).

13. Capteur (1) selon l'une quelconque des revendications précédentes, **caractérisé en ce que** les surfaces supérieures (360, 260) des porte-à-faux de référence et de test (3, 2) sont activées par une couche d'activation (340, 240),

la couche d'activation (340, 240) étant conçue pour fournir une tension superficielle supérieure par rapport à la surface inférieure non activée (362, 262) des porte-à-faux de référence et de test (3, 2), et

la couche d'activation (340, 240) comprenant de l'or.

14. Capteur (1) selon l'une quelconque des revendications précédentes, **caractérisé en ce que** les surfaces supérieures ou inférieures (362, 262) des porte-à-faux de référence et de test (3, 2) sont passivées par une couche de passivation (344, 244),

la couche de passivation (344, 244) étant conçue pour minimiser une adhérence protéique non spécifique sur les porte-à-faux de référence et de test (3, 2), et

la couche de passivation (344, 244) comprenant du triméthoxysilane et/ou une substance de blocage.

15. Capteur (1) selon l'une quelconque des revendications précédentes, **caractérisé en ce que** les porte-à-faux de référence et de test (3, 2) présentent une autre couche (341, 241) qui comprend une monocouche auto-assemblée.

Fig. 1

EP 4 328 579 B1

Fig. 2A

Fig. 2B

EP 4 328 579 B1

Fig. 2C

200

220

N

260

22

262

Fig. 2D

200

220

N

260

22

262

Fig. 2E

200

220

260

F, D

22

262

Fig. 2F

200

220

260

F, D

22

262

EP 4 328 579 B1

Fig. 3A

22

20

z

y

x

Fig. 3B

Dq

Dl

EP 4 328 579 B1

Fig. 3C

Fig. 3D

EP 4 328 579 B1

Fig. 4A

EP 4 328 579 B1

Fig. 4B

Fig. 5A

Fig. 5B

Fig. 6

EP 4 328 579 B1

**EP 4 328 579 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007088018 A1
- DE 60023917 T2
- WO 9709584 A
- WO 2005100965 A1

**In der Beschreibung aufgeführte Nicht-Patentliteratur**

- **RASMUSSEN, P. A.** ; **HANSEN, O.** ; **BOISEN, A.** Cantilever surface stress sensors with single-crystalline silicon piezoresistors. *Applied Physics Letters*, 2005, vol. 86 (20), 203502, https://doi.org/10.1063/1.1900299